(19) Europäisches Patentamt
European Patent Office
Office européen des brevets

(11) Publication number: **0 209 237 A2**

(12) **EUROPEAN PATENT APPLICATION**

(21) Application number: **86304319.6**

(22) Date of filing: **06.06.86**

(51) Int. Cl.⁴: **C 07 D 499/64**, C 07 D 499/80, C 07 D 501/20, C 07 D 501/58, C 07 D 498/04, C 07 D 471/04, C 07 C 131/00, C 07 D 333/24, C 07 C 101/28, A 61 K 31/43, A 61 K 31/545
//
(C07D498/04, 265:00, 205:00), (C07D471/04, 221:00, 205:00)

(30) Priority: **11.06.85 GB 8514679**
**13.08.85 GB 8520255**

(43) Date of publication of application: **21.01.87**
**Bulletin 87/4**

(84) Designated Contracting States: **AT BE CH DE FR GB IT LI LU NL SE**

(71) Applicant: **BEECHAM GROUP PLC, Beecham House Great West Road, Brentford Middlesex TW8 9BD (GB)**

(72) Inventor: **Burton, George, 14 Onslow Gardens, Wallington Surrey SM6 9QN (GB)**
Inventor: **Pearson, Michael John, 26 Comptons Lane, Horsham West Sussex RH13 5NY (GB)**

(74) Representative: **Lockwood, Barbara Ann et al, Beecham Pharmaceuticals Biosciences Research Centre Great Burgh Yew Tree Bottom Road, Epsom Surrey KT18 5XQ (GB)**

(54) **Vinyl-glycyl-amino-beta-lactam derivatives.**

(57) β-Lactam antibiotics are disclosed having the partial structure:

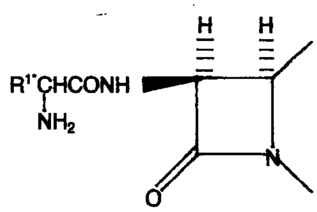

where $R^1$ is:

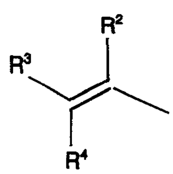

in which $R^2$, $R^3$ and $R^4$ are the same or different and each is hydrogen; halogen; optionally substituted $C_{1-6}$ alkyl; optionally substituted $C_{3-7}$ cycloalkyl; optionally substituted $C_{2-6}$ alkenyl; optionally substituted $C_{2-6}$ alkynyl; amido; cyano; carboxy; formyl; $C_{1-6}$ alkoxycarbonyl; mono- or di-$(C_{1-6})$alkylamido; $C_{1-6}$ alkylcarbonyl; an aryl group; a heterocyclyl group; or $R^3$ and $R^4$ are joined together to form the residue of a carbocyclic ring having a total of from 3 to 7 carbon atoms in the ring; or $R^3$ and $R^4$ are joined together to form the residue of a heterocyclic ring in which $R^3$ and $R^4$ together may be represented by the formula $-(CH_2)_mX(CH_2)_n-$ in which X is oxygen, sulphur or a group $NR^5$ wherein $R^5$ is hydrogen, acyl, an amino-protecting group, or $C_{1-6}$ alkyl, m is 1 to 3 and n is 1 to 3; and also the use thereof.

Processes for the preparation of the compounds are disclosed together with intermediates for use therein.

EP 0 209 237 A2

0209237

B1856

TITLE MODIFIED
see front page

# NOVEL COMPOUNDS

This invention relates to a class of β-lactam derivatives which have antibacterial activity and are of value in the treatment of infections in animals, including mammals and especially humans. In particular the invention relates to a class of β-lactam derivatives with an acyl group in the side-chain derived from vinyl glycine or a substituted derivative thereof. The invention also relates to a process for the preparation of such compounds, and to pharmaceutical compositions comprising them.

The present invention provides a compound of formula (I) or a pharmaceutically acceptable salt or <u>in vivo</u> hydrolysable derivative thereof:

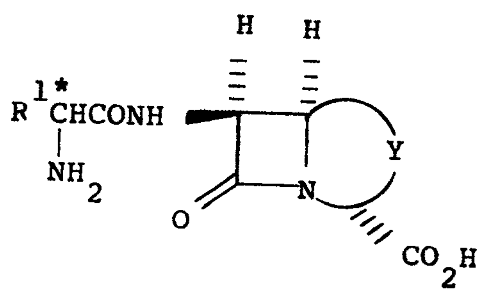

(I)

wherein $R^1$ represents a group:

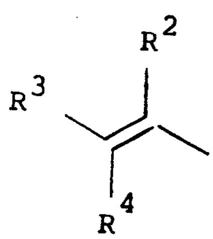

in which $R^2$, $R^3$ and $R^4$ are the same or different and each is hydrogen; halogen; optionally substituted $C_{1-6}$ alkyl; optionally substituted $C_{3-7}$ cycloalkyl; optionally substituted $C_{2-6}$ alkenyl; optionally substituted $C_{2-6}$ alkynyl; amido; cyano; carboxy; formyl; $C_{1-6}$ alkoxycarbonyl; mono- or di-$(C_{1-6})$alkylamido; $C_{1-6}$ alkylcarbonyl; an aryl group; a heterocyclyl group; or $R^3$ and $R^4$ are joined together to form the residue of a carbocyclic ring having a total of from 3 to 7 carbon atoms in the ring; or $R^3$ and $R^4$ are joined together to form the residue of a heterocyclic ring in which $R^3$ and $R^4$ together may be represented by the formula $-(CH_2)_mX(CH_2)_n-$ in which X is oxygen, sulphur or a group $NR^5$ wherein $R^5$ is hydrogen, acyl, an amino-protecting group, or $C_{1-6}$ alkyl, m is 1 to 3 and n is 1 to 3; and Y is:

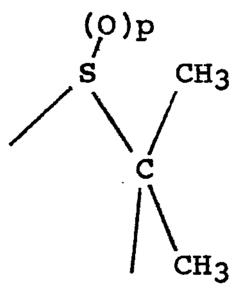

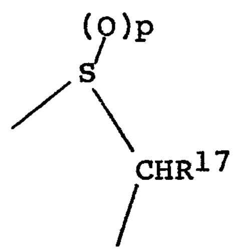

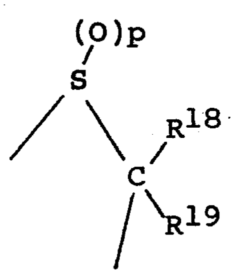

or

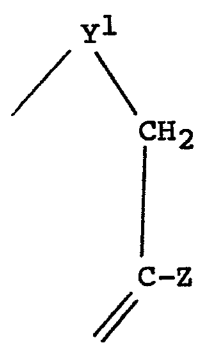

;

wherein $R^{17}$ is hydrogen or $C_{1-6}$ alkyl; $R^{18}$ and $R^{19}$ may be the same or different each being $C_{2-6}$ alkyl; p is an integer having the value 0, 1 or 2; $Y^1$ is oxygen, sulphur, SO, $SO_2$, or $-CH_2-$; and Z represents hydrogen,

halogen or an organic group such as $C_{1-4}$ alkoxy, $-CH_2Q$ or $-CH=CH-Q$ wherein Q represents hydrogen, halogen, hydroxy, mercapto, cyano, carboxy, carbamoyloxy, carboxylic ester, $C_{1-4}$ alkyloxy, acyloxy, aryl, a heterocyclyl group bonded via carbon, a heterocyclylthio group or a nitrogen containing heterocyclic group bonded via nitrogen; with the proviso that when Y is $-S-C(CH_3)_2-$ and $R^2$ is methyl, $R^3$ cannot be hydrogen when $R^4$ is phenyl and $R^3$ cannot be phenyl when $R^4$ is hydrogen.

Suitably Y is $-S-C(CH_3)_2-$, $-S-CH_2-$,
$-S-CH_2-C(CH_2Q)=$; or
$-O-CH_2-C(CH_2Q)=$.

Preferred values for Y in the compounds of formula (I) are $-S-C(CH_3)_2-$ and $-S-CH_2-C(CH_2Q)=$, i.e. the compound of formula (I) is a derivative of a penicillin or cephalosporin.

A particularly preferred value for Y is $-S-C(CH_3)_2-$.

Substituents that may be present on those groups $R^2$, $R^3$ and $R^4$ defined hereinabove as being optionally substituted include halogen, carboxy, $C_{1-6}$ alkoxycarbonyl, carbamoyl, aryl, heterocyclyl, hydroxy, $C_{1-6}$ alkanoyloxy, cyano, amino, $C_{1-6}$ alkanoylamino, mono- or di- $(C_{1-6})$ alkylamino, aryl $(C_{1-6})$ alkylthio, $C_{1-6}$ alkoxy, or $C_{3-7}$ cycloalkyl.

When used herein the term ''aryl'' includes phenyl and naphthyl optionally substituted with up to five fluorine, chlorine, $C_{1-6}$ alkyl, $C_{1-6}$ alkoxy, halo $(C_{1-6})$alkyl, hydroxy, amino, carboxy, $C_{1-6}$ alkoxycarbonyl, or $C_{1-6}$ alkoxycarbonyl-$(C_{1-6})$alkyl groups.

The term ''heterocyclyl'' herein denotes single or fused aromatic or non-aromatic heterocyclic rings linked via a carbon atom to the rest of the molecule, said rings containing up to four hetero atoms selected from oxygen, nitrogen and sulphur and being optionally substituted with up to three halogen, $C_{1-6}$ alkyl, $C_{1-6}$ alkoxy, halo-($C_{1-6}$)-alkyl, hydroxy, amino, carboxy, $C_{1-6}$ alkoxycarbonyl, $C_{1-6}$ alkoxycarbonyl($C_{1-6}$) alkyl, aryl or oxo groups. Suitably the heterocyclic ring comprises from 4 to 7 ring atoms, preferably 5 or 6 atoms. Depending on the nature of the heterocyclyl group, certain compounds within formula(I) may occur in two or more tautomeric forms; these are included within the scope of the present invention.

The term 'halogen' refers to fluorine, chlorine, bromine and iodine.

As used herein the term 'acyl' denotes $C_{1-6}$ alkylcarbonyl, arylcarbonyl or aryl $C_{1-6}$ alkylcarbonyl.

As used herein, the term 'amino-protecting group' denotes a group attached to nitrogen which may be readily cleaved to give a free amino group ($-NH_2$ or $-NH-$).

Such groups and conditions for their cleavage are well known in the art [see, for example, 'Protective groups in Organic Synthesis' by T.W. Greene (John Wiley and Sons, 1981)]. Examples of suitable amino-protecting groups include benzyloxycarbonyl optionally substituted in the phenyl ring by one or two substituents selected from $C_{1-4}$ alkyl, $C_{1-4}$ alkoxy, trifluoromethyl, halogen or nitro; $C_{1-4}$ alkoxycarbonyl, for example tert-butoxycarbonyl; or $C_{1-4}$ alkyloxycarbonyl optionally substituted in the alkyl group by up to

three substituents chosen from $C_{1-4}$ alkoxy, halo or nitro, for example 2,2,2,-trichloroethoxycarbonyl or 1-chloroethoxycarbonyl.

As used herein the term carboxylic acid ester includes $C_{1-6}$ alkyl, $C_{2-6}$ alkenyl, and aryl $C_{1-6}$ alkyl esters, and esters which may readily be cleaved such as in vivo hydrolysable ester groups as hereinbelow defined and other ester groups noted hereinbelow as being removable under conventional conditions.

The carbon atom marked * in formula (I) is asymmetric and the compound may be derived from the side-chain having a D, L or DL configuration at that position. All forms of compound (I) are included in this invention. Suitably, the carbon atom marked * is derived from the D-configuration; and compounds with that configuration are referred to herein as D-stereoisomers.

The term 'in vivo hydrolysable derivative' encompasses in vivo hydrolysable esters and other pro-drugs of the compound of formula (I).

Examples of suitable pharmaceutically acceptable in vivo hydrolysable ester groups include those which break down readily in the human body to leave the parent acid or its salt. Suitable ester groups of this type include those of part formula (i), (ii) and (iii):

$$-CO_2\overset{R^a}{\overset{|}{C}}H-O.CO.R^b \qquad (i)$$

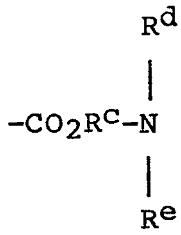 (ii)

$-CO_2CH_2OR^f$ (iii)

wherein $R^a$ is hydrogen, methyl, or phenyl, $R^b$ is $C_{1-6}$ alkyl, $C_{1-6}$ alkoxy or phenyl; or $R^a$ and $R^b$ together form a 1,2-phenylene group optionally substituted by one or two methoxy groups; $R^c$ represents $C_{1-6}$ alkylene optionally substituted with a methyl or ethyl group - $R^d$ and $R^e$ independently represent $C_{1-6}$ alkyl; $R^f$ represents $C_{1-6}$ alkyl. Examples of suitable in vivo hydrolysable ester group include for example acyloxyalkyl groups such as acetoxymethyl, pivaloyloxymethyl, α-acetoxyethyl and α-pivaloyloxyethyl groups; alkoxycarbonyloxyalkyl groups, such as ethoxycarbonyloxymethyl and α-ethoxycarbonyloxyethyl; dialkylaminoalkyl especially di-loweralkylamino alkyl groups such as dimethylaminomethyl, dimethylaminoethyl, diethylaminomethyl or diethylaminoethyl; lactone groups such as phthalidyl and dimethoxyphthalidyl; the group of formula:

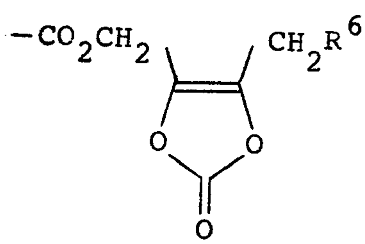

wherein $R^6$ is hydrogen, $C_{1-6}$ alkyl or phenyl; and esters linked to a second β-lactam antibiotic or to a β-lactamase inhibitor.

As will readily be appreciated by those skilled in the art the compound of the invention of formula (I) contain both an amino group and a CONH- moiety in the side-chain and will therefore form cyclic derivatives on reaction with aldehydes (such as formaldehyde) or ketones (such as acetone). Such cyclic derivatives are further examples of in vivo hydrolysable derivatives or pro-drugs of the compounds of formula (I).

Thus, in another aspect of the present invention, there is provided a pro-drug of a compound of formula (I) having the formula (Ia) or a pharmaceutically acceptable salt or in vivo hydrolysable ester thereof:

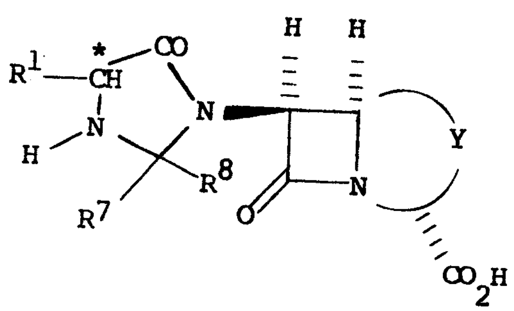

(Ia)

wherein $R^1$, * and Y are as defined for formula (I) above; and $R^7$ and $R^8$ are the same or different and each is hydrogen or $C_{1-6}$ alkyl. Preferred compounds within formula (Ia) include those in which $R^7$ and $R^8$ are both hydrogen or both methyl.

The compounds of the present invention contain both an amino group and a carboxyl group and may, therefore, exist as the zwitterion or may form salts with suitable acids or bases.

Suitable pharmaceutically acceptable salts of the compounds of formula (I) or (Ia) include metal salts, eg aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-β-phenethylamine, dehydroabietylamine, N,N'-bisdehydroabietylamine, ethylenediamine, or bases of the pyridine type such as pyridine, collidine or quinoline.

Suitable acid addition salts of compound (I) or (Ia) include pharmaceutically acceptable inorganic salts such as the sulphate, nitrate, phosphate, borate, hydrochloride and hydrobromide and pharmaceutically acceptable organic acid addition salts such as acetate, tartrate, maleate, citrate, succinate, benzoate, ascorbate, methane-sulphate, α-keto glutarate, α-glycerophosphate, and glucose-1-phosphate. Preferably the acid addition salt is a hemisuccinate, hydrochloride, α-ketoglutarate, α-glycerophosphate or glucose-1-phosphate, in particular the hydrochloride salt.

Salts within formula (I) or (Ia) may be prepared by salt exchange in conventional manner, for example a solution of the lithium salt in water may be passed through a bed of ion exchange resin in the sodium form (eg Amberlite IR 120; a sodium salt of a sulphonated polystyrene divinyl benzene co-polymer) in about ten-fold excess until elution is complete; the resulting sodium salt may be obtained by freeze drying

or the like. Similarly a sodium salt may be converted to a lithium salt or to a potassium salt in similar manner. (N.B. Amberlite is a trade name).

Suitable $C_{1-6}$ alkyl groups for $R^2$, $R^3$ and $R^4$ may be straight or branched chain and include methyl, ethyl, n or iso-propyl, n-, sec-, iso- or tert-butyl. In those cases where the $C_{1-6}$ alkyl group carries a substituent the preferred $C_{1-6}$ alkyl groups for $R^2$, $R^3$ and $R^4$ include methyl, ethyl and n-propyl.

Suitable $C_{3-7}$ cycloalkyl groups for $R^2$, $R^3$ and $R^4$ include cyclopropyl, cyclobutyl, cyclopentyl and cyclohexyl.

Suitable $C_{2-6}$ alkenyl groups for $R^2$, $R^3$ and $R^4$ include ethenyl, propenyl and butenyl.

Suitable $C_{2-6}$ alkynyl groups for $R^2$, $R^3$ and $R^4$ include ethynyl, propynyl and butynyl.

Suitably when $R^3$ and $R^4$ are joined together to form a carbocyclic ring, the carbocyclic ring contains a total of 3,4, 5 or 6 carbon atoms in the ring. The carbocyclic ring may be fully saturated or may contain up to two conjugated or non-conjugated double bonds.

Suitably when $R^3$ and $R^4$ are joined together to form a heterocyclic ring one of the integers n and m has the value of 2.

Preferably n and m are both 2.

Suitable aryl groups for $R^2$, $R^3$ and $R^4$ include phenyl, optionally mono- or di-substituted by $C_{1-6}$ alkyl,

hydroxy, $C_{1-6}$ alkoxy, trifluoromethyl, chlorine or fluorine.

Suitable heterocyclyl groups for $R^2$, $R^3$ and $R^4$ include 2-thienyl, 3-thienyl, 2-furyl and 3-furyl.

More suitably $R^2$ is hydrogen, methyl, ethyl, hydroxymethyl, methoxymethyl, phenyl or 2-thienyl.

More suitably $R^3$ is hydrogen, methyl, acetoxymethyl, ethyl, vinyl, cyclohexyl, 4-chlorophenyl, phenyl, or chloro.

More suitably $R^4$ is hydrogen, methyl, or chloro.

One preferred subgroup within the present invention provides a compound of formula (II) or a pharmaceutically acceptable salt or *in vivo* hydrolysable ester thereof:

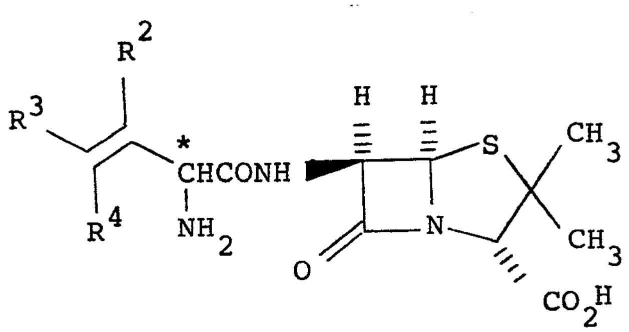

(II)

wherein $R^2$, $R^3$, $R^4$ and * are as hereinbefore defined.

Preferred groups for $R^2$ include hydrogen, phenyl, methyl and ethyl.

Preferred groups for $R^3$ and $R^4$ include hydrogen, ethyl, and chlorine.

In one preferred aspect of the invention, $R^2$, $R^3$ and $R^4$ are all hydrogen.

In a particularly preferred aspect of the invention, $R^2$ is hydrogen and $R^3$ and $R^4$ are both chlorine.

In another particularly preferred aspect of the invention $R^3$ is ethyl and $R^2$ and $R^4$ are both hydrogen.

In yet a further particularly preferred aspect of the invention $R^2$ is hydrogen and $R^3$ and $R^4$ together form a carbocylic ring having a total of 6 carbon atoms.

Since the β-lactam antibiotic compounds of the present invention are intended for use in pharmaceutical compositions it will readily be understood that they are each provided in substantially pure form, for example at least 50% pure, more suitably at least 75% pure and preferably at least 95% pure (% are on a wt/wt basis). Impure preparations of the compounds may be used for preparing the more pure forms used in the pharmaceutical compositions. Although the purity of intermediate compounds of the present invention is less critical it will readily be understood that the substantially pure form is preferred as for the β-lactam antibiotic compounds. Preferably, whenever possible, the compounds of the present invention are obtained in crystalline form.

Some of the compounds of this invention may be crystallised or recrystallised from solvents containing water. In such cases water of hydration may be formed. This invention includes within its scope stoichiometric hydrates as well as compounds containing variable amounts of water that may be produced by processes such as lyophilisation.

Suitable values for Q in the compounds of the formula (I) or (Ia) include the acetoxy, heterocyclylthio group, and nitrogen containing heterocyclic group bonded via nitrogen.

When the group Z as hereinbefore defined is -CH = CH-Q, preferred values of Q include methyl and 4-methyl-1,3-thiazol-5-yl.

The heterocyclylthio group may suitably be represented by the formula:

- S - Het

wherein 'Het' is a five or six membered heterocyclic ring containing from 1 to 4 atoms selected from N, O, and S unsubstituted or substituted with one or two groups selected from $C_{1-6}$ alkyl, $C_{1-6}$ alkoxy, aminoalkyl, hydroxyalkyl, $C_{1-6}$ alkenyl, alkoxyalkyl, carboxyalkyl, sulphonylalkyl, carbamoylalkyl, trifluoromethyl, hydroxy, halogen, oxo, mono- and di-($C_{1-6}$)alkylaminoalkyl, and carboxyalkyl or two substituents may be linked to form the residue of a heterocyclic or carbocyclic ring.

Examples of the group 'Het' include unsubstituted and substituted imidazolyl, triazolyl, tetrazolyl, thiazolyl, thiadiazolyl, thiatriazolyl, oxazolyl, triazinyl and oxadiazolyl.

Suitable groups 'Het' include unsubstituted and substituted 1, 2, 3-triazolyl; 1, 2, 4-triazolyl; tetrazolyl; oxazolyl; thiazolyl; 1, 3, 4-oxadiazolyl; 1, 3, 4-thiadiazolyl, or 1, 2, 4-thiadiazolyl.
The heterocyclylthio group may thus be, for example, 1-methyl-1H-tetrazol-5-ylthio, 2-methyl-1,3,4-thia-

diazol-5-ylthio, 1-carboxymethyl-1H-tetrazol-5-ylthio or 6-hydroxy-2-methyl-5-oxo-2H-1,2,4-triazin-3-ylthio.

A preferred heterocyclylthio group when the group Z as hereinbefore defined is $CH_2Q$ is 1,2,3-triazol-4-ylthio of structure:

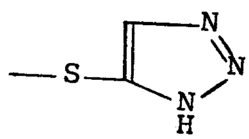

The nitrogen-containing heterocyclic group bonded <u>via</u> nitrogen is suitably an optionally substituted pyridinium group suitably the pyridinium group is substituted with one or two groups selected from $C_{1-6}$ alkyl, $C_{1-6}$ alkoxy, hydroxyalkyl, $C_{1-6}$ alkenyl, alkoxyalkyl, carboxyalkyl, sulphonylalkyl, carbamoylmethyl, carbamoyl, trifluoromethyl, hydroxy, halogen, oxo, and aminoalkyl.

More suitably, the nitrogen-containing heterocyclic group bonded <u>via</u> nitrogen may be an optionally substituted tetrazolyl group, particularly 5-methyltetrazol-2-yl of structure:

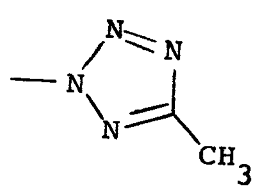

Specific compounds within the invention include the following or a pharmaceutically acceptable salt or in vivo hydrolysable ester thereof:

6β-(D-2-Aminobut-3-enamido)penicillanic acid

6β-(D-2-Amino-3-phenylbut-3-enamido)penicillanic acid

6β-(D-2-Amino-4,4-dichlorobut-3-enamido) penicillanic acid

6β-(D-2-Amino-3-methylbut-3-enamido)penicillanic acid

7β-(D-2-Amino-3-methylbut-3-enamido) cephalosporanic acid

7β-[D-2-Amino-3-methylbut-3-enamido]-3-desacetoxy cephalosporanic acid

6β-(D-2-Amino-3-ethylbut-3-enamido) penicillanic acid

6β-(D-2-Amino-3-methylpent-3(E)-enamido)-penicillanic acid

6β-(D-2-Amino-pent-3(E)-enamido)penicillanic acid

6β-(D-2-Amino-3-methylhex-3(E),5-dienamido)-penicillanic acid

6β-(D-2-Aminohex-3(E)-enamido)penicillanic acid

6β-(D-2-Amino-4-cyclohexylbut-3(E)-enamido)-penicillanic acid

6β-(D-2-Amino-4-phenylbut-3(E)-enamido)-
penicillanic acid

6β-[D,L-2-Amino-4-(4-chlorophenyl)but-3(E)enamido]
penicillanic acid

6,β-(D-2-Amino-3-trifluoromethylbut-3-enamido)-
penicillanic acid

6β-(D-2-Amino-3-thien-2-ylbut-3-enamido)-
penicillanic acid

6β-(D,L-2-Amino-4-chloro-3-thien-2-ylbut-3(Z)-
enamido)penicillanic acid

6B-(D,L-2-Amino-4-chloro-3-thien-2-ylbut-3(E)-
enamido)penicillanic acid

6β-(D-2-Amino-4-chloro-3-methylbut-3(E)-enamido)-
penicillanic acid

6β-(D,L-2-Amino-4-chloro-3-methylbut-3(Z)-enamido)
penicillanic acid

6β-[5-Acetoxy-D-2-aminopent-3(E)-enamido]-
penicillanic acid

6β-(D-2-Amino-3-cyclohexylidenepropanamido)
penicillanic acid

6β-(D,L-2-Amino-4-methoxy-3-methylenebutanamido)
penicillanic acid

6β-(D-2-Amino-4-methylpent-3-enamido)penicillanic
acid

The compounds of formula (I) may be prepared by reacting a compound of formula (III):

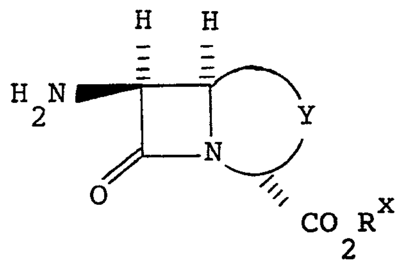

(III)

wherein the amino group is optionally substituted with a group which permits acylation to take place, and $R^x$ is hydrogen or a carboxyl-blocking group, with an N-acylating compound formed from a salt or N-protected derivative of an amino acid of formula (IV):

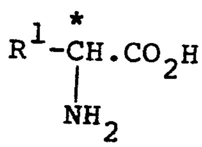

(IV)

wherein $R^1$ and * are as defined with respect to formula (I) above and any reactive groups may be protected; and thereafter, if necessary, carrying out one or more of the following steps:

(i) removing any carboxyl-blocking groups $R^x$;

(ii) removing any protecting groups elsewhere in in the molecule;

(iii) converting the product into a salt or *in vivo* hydrolysable derivative thereof.

The *in vivo* hydrolysable derivatives (pro-drugs) of formula (Ia) may be prepared by conventional methodology, i.e. reacting a compound of formula (I) or a pharmaceutically acceptable salt or *in vivo* hydrolysable ester thereof with an aldehyde or ketone of the formula:

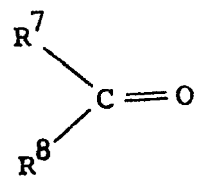

wherein $R^7$ and $R^8$ are as hereinbefore defined.

The compounds of formula (I) or (Ia) with the preferred D-side-chain stereochemistry may be separated from a mixture of diastereoisomers of formula (I) or (Ia) by conventional methods or prepared from amino acids of formula (IV) that have the D-configuration.

The amino group in the α-amino acid of formula (IV) is generally protected by a removable blocking group before the reactive *N*-acylating derivative is formed. Alternatively, the amino group in the amino acid (IV) may be protected by the prior formation of an acid addition salt, for example the hydrochloride.

Suitable groups which permit acylation to take place and which are optionally present on the amino group of the starting material of the formula (III) include N-silyl, N-stannyl and N-phosphorus groups, for example trialkylsilyl groups such as trimethylsilyl, trialkyltin groups such as tri-n-butyltin, groups of formula $-P.R^9R^{10}$ wherein $R^9$ is an alkyl, haloalkyl,

aryl, aralkyl, alkoxy, haloalkyl, aryl, aralkyl, alkoxy, haloalkoxy, aryloxy, aralkyloxy or dialkylamino group, $R^{10}$ is the same as $R^9$ or is halogen or $R^9$ and $R^{10}$ together form a ring; suitable such phosphorus groups being $-P(OC_2H_5)_2$, $-P(C_2H_5)_2$,

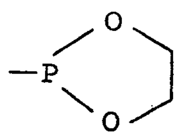 and 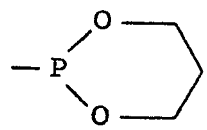

Suitable amino-blocking groups which may be used to block the amino group in (IV) are those amino-protecting groups well-known in the art which may be removed under conventional conditions without disruption of the remainder of the molecule.

Particularly suitable examples of amino-protecting groups which may be employed in derivatives of (IV) in which the amino group is blocked include 2,2,2-trichloroethyloxycarbonyl and 3-methoxycarbonylprop-2-en-2-yl.

Suitable carboxyl-blocking derivatives for the group $-CO_2R^x$ in formula (III) include salts and ester derivatives of the carboxylic acid. The derivative is preferably one which may readily be cleaved at a later stage of the reaction. Suitable salts include metal salts, such as those with sodium, potassium and lithium, and tertiary amine salts, such as those with trilower-alkylamines, N-ethylpiperidine, 2,6-lutidine, pyridine, N-methylpyrrolidine, dimethylpiperazine. A preferred salt is with triethylamine.

Suitable ester-forming carboxyl-blocking groups are those which may be removed under conventional conditions. Such groups for $R^x$ include benzyl, p-methoxybenzyl, 2,4,6-trimethylbenzyl, 3,5-di-t-butyl-4-hydroxy-benzyl, benzoylmethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetrahydrofuran-2-yl, tetrahydropyran-2-yl, pentachlorophenyl, p-toluenesulphonylethyl, methoxymethyl, a silyl, stannyl or phosphorus-containing group, such as described above, an oxime radical of formula $-N=CHR^{11}$ where $R^{11}$ is aryl or heterocyclic, or an _in vivo_ hydrolysable ester radical such as defined above.

The carboxyl group may be regenerated from any of the above esters by usual methods appropriate to the particular $R^x$ group, for example, acid - and base - catalysed hydrolysis, or by enzymically - catalysed hydrolysis, or by hydrogenation under conditions wherein other parts of the molecule are unaffected.

A reactive N-acylating derivative of a salt or N-protected derivative of the amino acid (IV) is employed in the above process. The choice of reactive derivative will of course be influenced by the chemical nature of the substituents of the acid.

Suitable N-acylating derivatives of salts or N-protected derivatives of the acid (IV) include acid halides, preferably the acid chloride or bromide. Acylation with an acid halide may be affected in the presence of an acid binding agent for example, tertiary amine (such as triethylamine or dimethylaniline), an inorganic base (such as calcium carbonate or sodium

bicarbonate) or an oxirane, which binds hydrogen halide liberated in the acylation reaction. The oxirane is preferably a $(C_{1-6})$-1,2,alkylene oxide - such as ethylene oxide or propylene oxide. The acylation reaction using an acid halide may be carried out at a temperature in the range -50°C to +50°C, preferably -20°C to +20°C, in aqueous or non-aqueous media such as aqueous acetone, aqueous tetrahydrofuran, ethyl acetate, dimethylacetamide, dimethylformamide, acetonitrile, dichloromethane, 1,2-dichloroethane, or mixtures thereof. Alternatively, the reaction may be carried out in an unstable emulsion of water-immiscible solvent, especially an aliphatic ester or ketone, such as methyl isobutyl ketone or butyl acetate.

The acid halide may be prepared by reacting salts or N-protected derivatives of the α-amino acid (IV) with a halogenating (eg chlorinating or brominating) agent such as phosphorus pentachloride, thionyl chloride or oxalyl chloride.

Alternatively, the N-acylating derivative of the salt or N-protected derivative of the amino acid (IV) may be a symmetrical or mixed anhydride. Suitable mixed anhydrides are alkoxyformic anhydrides, or anhydrides with, for example, carbonic acid monoesters, trimethyl acetic acid, thioacetic acid, diphenylacetic acid, benzoic acid, phosphorus acids (such as phosphoric or phosphorous acids) or aliphatic or aromatic sulphonic acids (such as p-toluenesulphonic acid). The reaction may be carried out in the presence of an organic base such as triethylamine.

Alternative N-acylating derivatives of salts or N-protected derivatives of acid (IV) are the acid azide, or activated esters such as esters with

2-mercaptopyridine, cyanomethanol, p-nitrophenol, 2,4-dinitrophenol, thiophenol, halophenols, including pentachlorophenol, monomethoxyphenol, N-hydroxy succinimide, N-hydroxy benzotriazole, or 8-hydroxyquinoline; or amides such as N-acylsaccharins or N-acylphthalimides; or an alkylidene iminoester prepared by reaction of the acid (IV) with an oxime.

Other reactive N-acylating derivatives of salts or N-protected derivatives of the acid (IV) include the reactive intermediates formed by reaction in situ with a condensing agent such as a carbodiimide, for example, N,N′-diethyl-, di -n-propyl- or diisopropylcarbodiimide, N,N'-di-cyclohexyl-carbodiimide, or N-ethyl-N'-3-dimethylaminopropyl-carbodiimide; a suitable carbonyl compound, for example, N,N'-carbonyldiimidazole or N,N'-carbonylditriazole; an isoxazolinium salt, for example, N-ethyl-5-phenylisoxazolinium-3-sulphonate or N-t-butyl-5-methylisoxazolinium perchlorate; or an N-alkoxycarbonyl 2-alkoxy-1,2-dihydroquinoline, such as N-ethoxycarbonyl 2-ethoxy-1,2-dihydroquinoline. Other condensing agents include Lewis acids; or a phosphoric acid condensing agent such as diethylphosphorylcyanide. The condensation reaction is preferably carried out in an organic reaction medium, for example, dichloromethane, dimethylformamide, acetonitrile, alcohol, benzene, dioxan or tetrahydrofuran.

Compounds of formula (IV) may be prepared by treating compounds of formula (IVA) or salts and esters thereof:

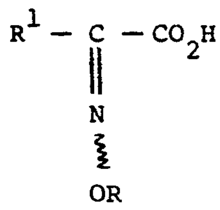

(IVA)

wherein $R^1$ is as hereinbefore defined and R is hydrogen, $C_{1-6}$ alkyl, $C_{2-6}$ alkenyl, $C_{3-7}$ cycloalkyl or aryl $C_{1-6}$ alkyl with a reducing agent which leaves the remainder of the molecule substantially unaffected; and thereafter, if necessary, converting the product into the free acid.

Suitable reducing agents include, for example, zinc in the presence of formic acid, and other reagents known in the art to be capable of converting oximes or oximino ethers into amines.

Compounds of the formula (IVA) are novel and form another aspect of the present invention.

It will be appreciated that compounds of the formula (IVA) may possess either syn or anti stereochemistry. Both syn- and anti stereoisomers and mixtures of the two stereoisomers in any proportion are encompassed within the present invention.

Preferably the group R in compounds of the formula (IVA) is methyl.

According to the invention there is also provided a process for the preparation of a compound of formula (IV) from a compound of formula (IVA) by the method hereinabove described.

The compound of formula (IVA) may also be written as (IVB):

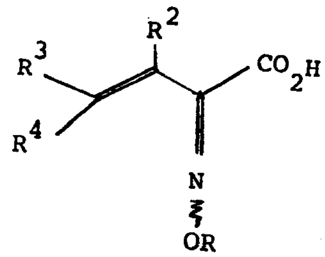

(IVB)

wherein R, $R^2$, $R^3$ and $R^4$ are as hereinbefore defined.

Compounds of formula (IVB) may be prepared by a variety of methods, for example via the Wittig or modified Wittig reactions shown in the general Scheme below in which R, $R^2$, $R^3$ and $R^4$ are as hereinbefore defined, $R^x$ is a readily removable carboxylic acid blocking group which may be removed at the end of the synthesis, Ar is an aryl group as hereinabove defined and $R^{20}$ is $C_{1-6}$ alkyl.

Scheme

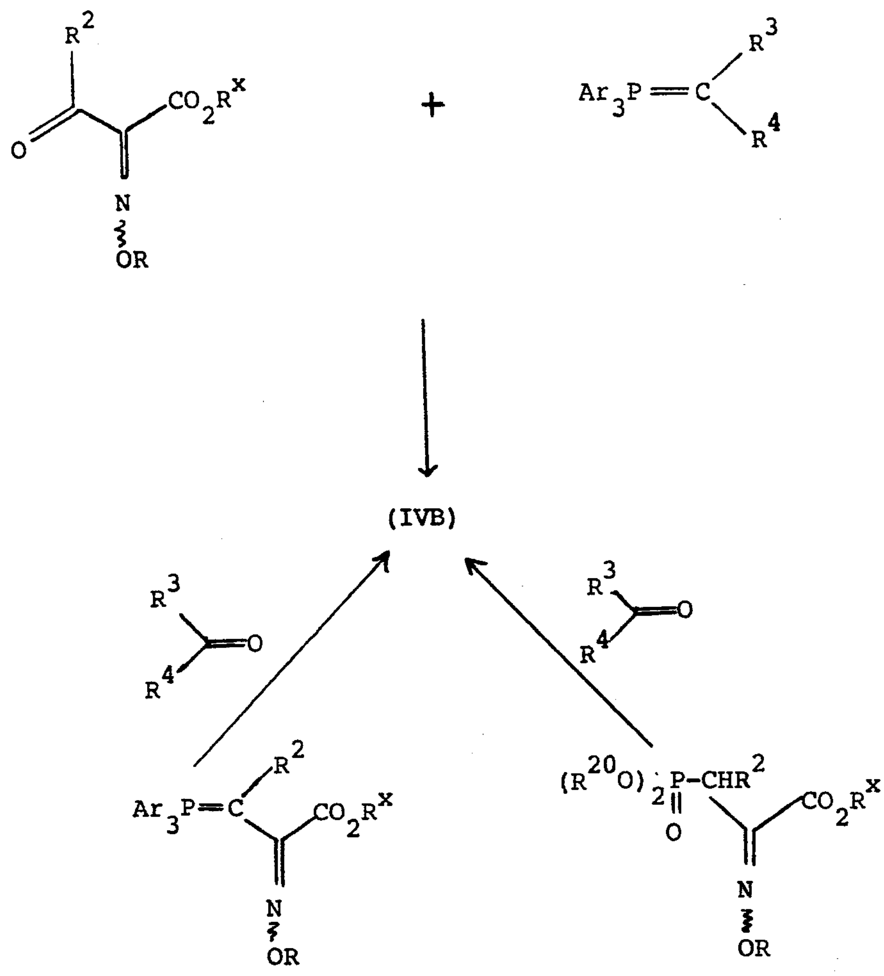


Details of the conditions and reagents used to effect the transformations shown in the Scheme above may be found in the Examples given hereinbelow.

Preferably the group Ar is phenyl and $R^{20}$ is $C_{1-4}$ alkyl especially methyl.

Compounds of the formula (IV) may also be prepared by deprotecting an N-protected derivative of a compound of formula (IV).

Useful intermediates of this type are those of formula (IVC):

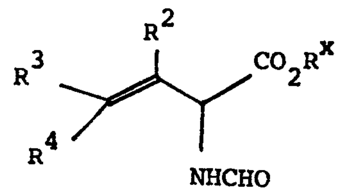

(IVC)

wherein $R^2$, $R^3$, $R^4$ and $R^x$ are as hereinbefore defined; which may be converted into compounds of formula (IV) by treatment with aqueous mineral acid, for example 5N hydrochloric acid; and thereafter, if necessary, removing the group $R^x$.

Compounds of formula (IVC) may be prepared by reaction of the appropriately substituted 2-oxazoline with activated zinc dust in a suitable solvent such as tetrahydrofuran, by a method analogous to that described by F.Heinzer and D.Bellus, Helv. Chem. Acta., 1981, 64, 2279.

The sub-group of compounds within the present invention of formula (V):

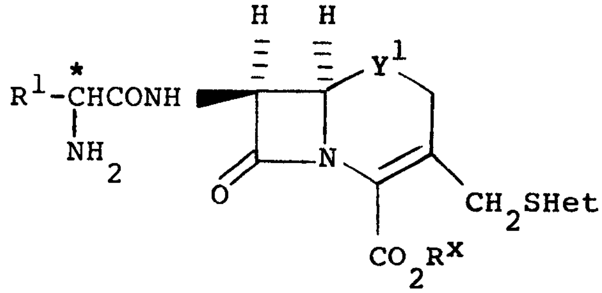

(V)

wherein $Y^1$, 'Het', $R^1$, and $R^x$ are as defined hereinbefore may suitably be prepared by reacting a compound of formula (VI):

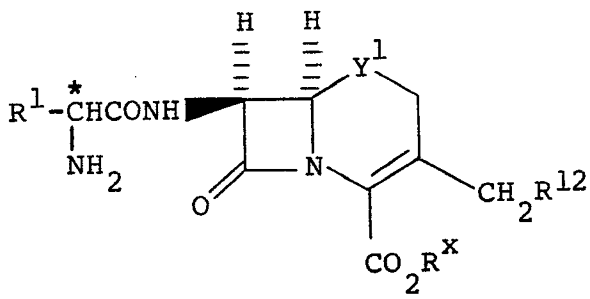

(VI)

wherein $Y^1$, $R^1$, $R^x$ and * are as hereinbefore defined and wherein any reactive groups may be protected and $R^{12}$ is a leaving group; with a thiol of formula:

Het SH

Suitable leaving groups $R^{12}$ include halogen such as iodide or bromide, acyloxy groups such as, for example, the acetoxy group, or aryl- or $C_{1-6}$ alkyl-sulphonyloxy groups such as p-toluenesulphonyloxy or methanesulphonyloxy.

The thiol HetSH may be reacted as the free compound or a salt with an alkali metal such as sodium or potassium. This reaction is desirably conducted in a solvent. For example, use can be made of water, or organic solvents inert to the starting compounds, such as dimethylformamide, dimethylacetamide, dioxane, acetone, alcohol, 1,2-dichloroethane, acetonitrile, dimethylsulfoxide or tetrahydrofuran, or mixtures thereof. The reaction temperature and time depend, among other factors, upon the starting compounds and

solvent to be employed but generally the reaction is carried out at a selected temperature within the range of 0 to 100°C for a selected time of a few hours to several days. The reaction is desirably conducted between pH 3 and 7.

To prevent oxidation of the thio compounds it is advantageous to carry out the reaction in an inert gaseous atmosphere, e.g. nitrogen gas.

The subgroup of compounds within the present invention of formula (VII).

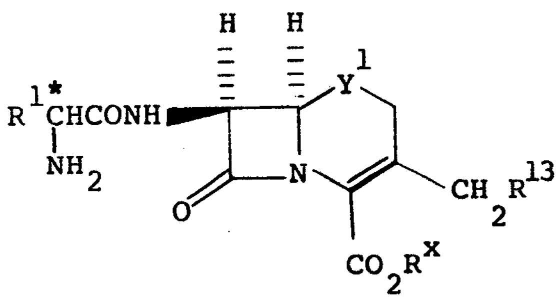

(VII)

wherein $R^1$, $Y^1$, $R^x$ and * are as defined hereinbefore and $R^{13}$ is an optionally substituted pyridinum group may suitably be prepared by reacting a compound of formula (VI) as hereinbefore defined with the appropriately substituted pyridine.

Suitably the reaction with the pyridine is carried out in a polar solvent such as water, and in the presence of a catalyst such as an alkali metal thiocyanate or an alkali metal halide such as, for example sodium iodide.

The antibiotic compounds according to the invention may be formulated for administration in any convenient way for use in human or veterinary medicine, by analogy with other antibiotics, and the invention therefore

includes within its scope a pharmaceutical composition comprising a compound of formula (I) or a pharmaceutically acceptable salt or in vivo hydrolysable derivative thereof together with a pharmaceutical carrier or excipient.

The composition may be formulated for administration by any route, such as oral, topical or parenteral. Administration by the oral route is generally preferred. The compositions may be in the form of tablets, capsules, powders, granules, lozenges, creams or liquid preparations, such as oral or sterile parenteral solutions or suspensions.

Tablets and capsules for administration may be in unit dose presentation form, and may contain conventional excipients such as binding agents, for example syrup, acacia, gelatin, sorbitol, tragacanth, or polyvinylpyrollidone: fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin,

sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parental suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The composition may contain from 0.1% to 99.5% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 mg to 20 g per day for an average adult patient (70 kg.), for instance 1500 mg per day, depending on the route and frequency of administration. Such dosages correspond to approximately 1.5 to 285 mg/kg per day. Suitably the dosage is from 0.25g to 6g per day.

The daily dosage is suitably given by administering a compound of the invention several times in a 24-hour period. Typically, 250 mg. is administered 2 or 3 times a day although, in practice, the dosage and frequency of administration which will be most suitable for an individual patient will vary with the age, weight and response of the patients, and there will be occasions when the physician will choose a higher or lower dosage and a different frequency of administration. Such dosage regimens are within the scope of this invention.

No toxicological effects are indicated when a compound of the invention of formula (I) or a pharmaceutically acceptable salt or in vivo hydrolysable derivative thereof is administered in the above mentioned dosage range.

The compound of formula (I) or a pharmaceutically acceptable salt or in vivo hydrolysable derivative thereof may be the sole therapeutic agent in the compositions of the invention or a combination with other antibiotics or with a β-lactamase inhibitor may be employed.

Advantageously, the compositions also comprise a compound of formula (VIII) or a pharmaceutically acceptable salt or ester thereof:

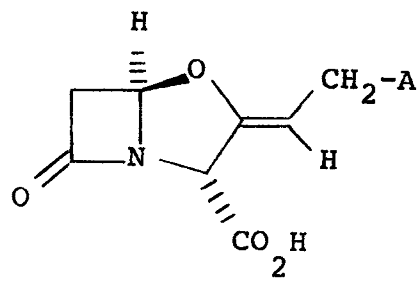

(VIII)

wherein A is hydroxyl; substituted hydroxyl; thiol; a group of formula $SO_2R^{14}$ wherein $R^{14}$ is $C_{1-6}$ alkyl; substituted thiol; amino; mono- or di-hydrocarbyl-substituted amino; mono- or di-acylamino; an optionally substituted triazolyl group; or an optionally substituted tetrazolyl group as described in EP 0 053 893.

Suitable substituents A in formula (VIII) include hydroxyl, tetrazol-1-yl and tetrazol-2-yl.

A further advantageous composition comprises a compound of formula (I) or a pharmaceutically acceptable salt or <u>in vivo</u> hydrolysable derivative thereof together with a compound of formula (IX) or a pharmaceutically acceptable salt or <u>in vivo</u> hydrolysable ester thereof:

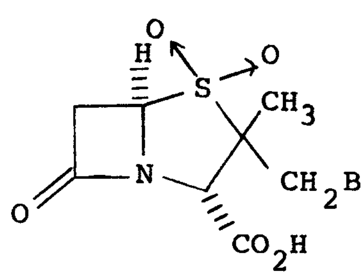

(IX)

wherein B is hydrogen, halogen or a group of formula:

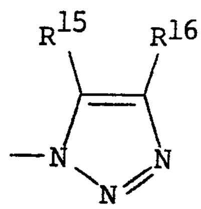

in which $R^{15}$ and $R^{16}$ are the same or different and each is hydrogen, $C_{1-6}$ alkoxycarbonyl, or carboxy or a pharmaceutically acceptable salt thereof.

Further suitable β-lactamase inhibitors include 6-alkylidene penems as described in European Patent Application No. 81301683.9 (Publication Number 0 041 768).

Further suitable β-lactamase inhibitors include 6β-bromopenicillanic acid and salts and in vivo hydrolysable esters thereof and β-iodo penicillanic acid and salts and in vivo hydrolysable esters thereof.

Compositions of this invention comprising a β-lactamase inhibitor are formulated in a conventional manner.

The present invention also provides a method of treating bacterial infections in animals, in particular humans or domestic mammals, which comprises the administration of a composition of this invention. The preferred method of administration is via the oral route.

The antibiotic compounds of the present invention are active against a wide range of Gram-positive organisms including B. subtilis; S. aureus such as, for example

Oxford; B. catarrhalis; S. faecalis I; S. pyogenes CN10; H. influenzae; E. coli; and P. mirabilis.

A particular feature of the compounds of the present invention of formula (I) and pharmaceutically acceptable salts and in vivo hydrolysable derivatives thereof is their excellent absorption as shown in animal species following oral administration.

The excellent absorption properties of these compounds may also be demonstrated by measuring the Absorption Index [that is the ratio of urine recoveries from oral (p.o.) and subcutaneous (s.c.) administration in animal species] which is generally as good as, and in some cases much better than, that of well known antibacterial agents such as amoxycillin.

The following Examples illustrate the preparation and biological activity of the compounds of the present invention.

The following abbreviations are used:

| Abbreviation | Full Name |
|---|---|
| DMF | Dimethylformamide |
| 6-APA | 6β-Aminopenicillanic acid |
| THF | Tetrahydrofuran |
| 7-ACA | 7β-Aminocephalosporanic acid |
| 7-ADCA | 7β-Amino-3-desacetoxycephalosporanic acid |
| NOE | Nuclear Overhauser Effect |

EXAMPLE 1

a. Potassium D-2-(3-methoxycarbonylprop-2-en-2-ylamino)but-3-enoate

Methyl acetoacetate (0.61ml, 5.6mmol) and D-2-aminobut-3-enoic acid (0.515g, 5.1mmol) in 0.87M potassium hydroxide in methanol (6.2 ml, 5.35mmol) were heated under reflux for 40 minutes and evaporated to dryness *in vacuo*. Toluene (10ml) and ethanol (5ml) were added to the residue then reevaporated and the residue triturated with ether to give the title compound (1.140g, 86%); $\nu_{max}$ (KBr) 1645, 1610, 1565, 1500, 1436, 1369, 1269, 1159, 925 and 784cm$^{-1}$; $\delta_H$(250MHz, DMSO-$d_6$) 1.77 (3H, s,), 3.47 (3H, s), 4.17 (1H, dd, $J_{H2, NH}$ 6.6Hz, $J_{H2, H3}$4.7Hz), 4.25 (1H, s), 4.8-5.0 (2H, m), 5.95-6.15 (1H, m), and 9.10 (1H, d, $J_{NH, H2}$6.6Hz).

b. 6β-(D-2-Aminobut-3-enamido)penicillanic acid

Potassium D-2-(3-methoxycarbonylprop-2-en-2ylamino)but-3-enoate (0.474g, 2mmol) in acetone (8ml) was cooled to -20°C then N-methylmorpholine (2µl) and ethyl chloro formate (192µl) were added and the mixture stirred at -20 ± 5°C for 40 minutes. A solution of 6β-amino penicillanic acid (0.48g, 2.2mmol) and triethylamine (320µl) in acetone (8ml) and water (16ml) was added and the solution stirred for one hour without cooling. Acetone was removed *in vacuo* and the aqueous residue maintained at pH1 with 5N hydrochloric acid for 20 minutes, washed with ether (2 x 25ml), adjusted to pH 4.7 and freeze dried (1.24g). Chromatography on HP20SS eluting with water afforded the title compound (0.104g, 17%); $\nu_{max}$ (KBr) 1767, 1695, 1591, 1534, 1393, 1388, 1255 and 1122cm$^{-1}$; $\delta_H$(250MHz, $D_2O$) 1,53 (3H, s),

1.64 (3H, s), 4.26 (1H, s), 4.69 (1H, d, J 7.5Hz), 5.54 (1H, d, J 3.8Hz), 5.58 (1H, d, J 3.8Hz), 5.6-5.7 (2H, m), and 5.9-6.1 (1H, m). [Mass spectrum: +ve ion (glycerol/$H_2O$) $MH^+$ (300)].

EXAMPLE 2

a. D-2-(2,2,2-Trichloroethoxycarbonylamino)-but-3-enoic acid

2,2,2-Trichloroethyl chloroformate (0.56ml, 4.1mmol) in acetone (10ml) was added dropwise to D-2-aminobut-3-enoic acid (0.373g, 3.7mmol) in water (20ml) and acetone (10ml) maintained at pH 9.8 ± 0.2 with 2.5N NaOH. The solution was then stirred for 30 minutes, concentrated *in vacuo*, washed with ether (2x30ml), acidified to pH2 and extracted with chloroform (4x25ml). The extracts were washed with water (2x50ml), dried and evaporated to provide the title compound as a crystalline solid, recrystallised from ethyl acetate-cyclohexane (0.741g, 73%), mp. 141-142°C; $[\alpha]^{20}_D$ -3.16° (c 1, EtOH); $\delta_H$(60MHz, $(CD_3)_2CO$) 4.72 (2H, s), 4.7-5.1 (1H, m), 5.1-5.5 (2H, m), 5.7-6.3 (1H,m), 7.05 (1H,d, $J_{NH,\ H-3}$7Hz), and 9.41 (1H, br s).

b. Sodium 6β-[D-2(2,2,2-trichloroethoxycarbonylamino)but-3-enamido]penicillanate

A suspension of D-2-(2,2,2-trichloroethoxycarbonylamino)but-3-enoic acid (0.639g, 2.5mmol) in dichloromethane (20ml) was treated with oxalyl chloride (0.5ml) and DMF (5μl) and stirred until a clear solution was obtained, ca. 1hour. The solvent was removed *in vacuo* and the acid chloride, $\nu_{max}$ ($CH_2Cl_2$) 1800, 1750 and 1502$cm^{-1}$, in acetone (10ml) added to

6-APA (0.648g, 3mmol) and triethylamine (850µl) in water (20ml) and acetone (10ml). After the solution had been stirred for one hour it was concentrated *in vacuo*, washed with ether (2x25ml), acidified to pH2.5 and extracted with chloroform (4 x 25ml). The extracts were washed with water (2 x 50ml), dried and evaporated to a foam (1.099g). This in ether (20ml) was treated with 2N sodium 2-ethyl hexanoate in 4-methylpentan-2-one (1.16ml), diluted with light petroleum, bp. 60-80°C, (20ml) and the precipitated sodium salt collected and dried in vacuo (0.996g, 80%).

c. 6β-(D-2-Aminobut-3-enamido)penicillanic acid

Freshly activated zinc dust (1.5g) was added to sodium 6β-[D-2-(2,2,2-trichloroethoxycarbonylamino)but-3-enamido]penicillanate (0.776g) in 0.25M potassium dihydrogen phosphate (16ml) and THF (4ml) and the mixture maintained at pH5 with 40% orthophosphoric acid. After 10 minutes a further portion of activated zinc (1.5g) was added and stirring continued for 10 minutes when reverse phase hplc showed no remaining starting material. The zinc was filtered off, filtrate washed with ether (2 x 25ml), concentrated *in vacuo* and chromatographed on HP20SS eluting with water. Fractions containing the product were bulked, reduced to *ca* 2ml, diluted with n-propanol (4ml) and left to crystallise at 0°C. The crystalline dihydrate was collected and dried *in vacuo* (0.171g, 33%); (Found: C,43.22; H,6.02; N,12.44; S,9.86%. $C_{12}H_{17}N_3O_4S$. $2H_2O$ requires: C,42.97; H,6.31; N,12.53; S,9.56%); ir and nmr spectra as in example 1b.

EXAMPLE 3

6β-(D-2-Aminobut-3-enamido)penicillanic acid

Hydrogen chloride gas was bubbled through D-2-aminobut-3-enoic acid hydrochloride (0.138g, 1mmol) in methanol free dichloromethane (5ml) for 30 minutes. The mixture was cooled in an ice bath and freshly sublimed phosphorous pentachloride (0.21g, 1.005mmol) in dichloromethane (3ml) added. After 30 minutes the suspension was evaporated to dryness, toluene (5ml) added, reevaporated and the residue triturated with anhydrous ether. This acid chloride hydrochloride was suspended in dichloromethane (5ml), cooled to -30°C and treated with N,O-bistrimethylsilyl 6-APA (from 6-APA, 0.216g, 1mmol) in dichloromethane (10ml). The mixture was stirred without cooling, for two hours then extracted with water (2 x 10ml). The aqueous extract was adjusted to pH 4.8 and freeze dried (0.600g). Chromatography on HP20SS followed by preparative reverse phase hplc provided material identical with that described in example 1b.

Preparation 1

a. Methyl D-2-(benzyloxycarbonylamino)but-3-enoate

N-Benzyloxycarbonyl-D-glutamic acid 5-methyl ester (3.65g., 12.4mmol) and cupric acetate monohydrate (0.62g, 3.1mmol) in toluene (75ml) were stirred under argon at 20°C for 1hour. Acetic acid free lead tetra-acetate (11.05g, 24.8mmol) was added and the mixture stirred vigorously and heated at 100°C for 3 hours. The cooled solution was diluted with ethyl acetate (75ml), filtered through celite, washed with water (2 x 50ml) and saturated sodium hydrogen carbonate solution (2 x 50ml), dried and evaporated to

an oil (3.174g). Flash chromatography eluting with 10% ethyl acetate in hexane afforded the title compound, (0.974g, 32%); $\delta_H$(60MHz, $CDCl_3$) 3.65 (3H, s), 4.7-5.05 (1H, m), 5.08 (2H, s), 5.1-5.5 (2H, m), 5.5-5.7 (1H, m), 5.6-6.2 (3H, m), and 7.27 (5H, s).

b. D-2-Aminobut-3-enoic acid hydrochloride

Methyl D-2-(benzyloxycarbonylamino)but-3-enoate (2.30g) in 6N hydrochloric acid (45ml) was heated under reflux for one hour, cooled, washed with chloroform (2 x 25ml) and evaporated to dryness. The crystalline residue was triturated with hot acetone to give the title compound, (1.058g, 83%), mp 168-170°C dec.; $[\alpha]^{20}_D$ -63.64° (c 1.9, $H_2O$); $\delta_H$(250MHz, $D_2O$) 4.60 (1H, d, J 7Hz), 5.5-5.65 (2H, m), and 5.9-6.1 (1H, m).

c. D-2-Aminobut-3-enoic acid

Propylene oxide (1.35ml) was added to a stirred solution of the hydrochloride (0.929g) in methanol (4ml). After two hours more methanol (4ml) was added, the solid collected, washed with methanol (2ml) and dried in vacuo (0.562g, 82%), mp 218-222°C dec.; $[\alpha]^{20}_D$ -79.05° (c 1, $H_2O$); (Found C, 47.32; H, 6.84; N, 13.64%. $C_4H_7NO_2$ requires: C, 47.52, H, 6.98; N, 13.85%).

Example 4

a. D,L-3-Phenyl-2-(2,2,2-trichloroethoxycarbonylamino) but-3-enoic acid

D,L-2-Amino-3-phenylbut-3-enoic acid (0.514g, 2.9mmol, F. Heinzer and D. Bellus, Helv. Chim. Acta., 1981, 64, 2279) was converted to the title compound as described in example 2a (0.650g, 63%); mp 103-105°C from ethyl acetate-cyclohexane; (Found: C, 44.27; H, 3.38; N, 4.02; Cl, 30.14%. $C_{13}H_{12}Cl_3NO_4$ requires: C,

44.28; H, 3.43; N, 3.97; Cl, 30.16%); $\delta_H$(60MHz, $(CD_3)_2CO$) 4.73 (2H, s), 5.32 (1H, d, J 8Hz), 5.42 and 5.52 (2H, 2s), 6.60 (1H, br s), 6.73 (1H, d, J 8Hz), and 7.0-7.6 (5H, m).

b. Sodium 6β-[D,L-3-phenyl-2-(2,2,2-trichloroethoxy carbonylamino)but-3-enamido]penicillanate

Prepared as described in example 2b (78%).

c. 6β-(2-Amino-3-phenylbut-3-enamido)penicillanic acids

Deprotection of sodium 6β-[D,L-3-phenyl-2-(2,2,2-trichloroethoxycarbonylamino)but-3-enamido] penicillinate (0.43g) was performed as described in example 2c. Chromatography of the crude product on HP20SS eluting with 4% THF in water provided 6β-(L-2-amino-3-phenylbut-3-enamido)penicillanic acid (50mg, 17%); $\nu_{max}$ (KBr) 1769, 1676, 1602, 1512, 1397 and 1318cm$^{-1}$; $\delta_H$(250MHz, $D_2O$) 1.45 (3H, s), 1.46 (3H, s), 4.17 (1H, s), 4.93 (1H, s), 5.40 (1H, d, J 4Hz), 5.53 (1H, d, J 4Hz), 5.54 and 5.65 (2H, 2s), and 7.43 (5H, s); followed by a mixture of D and L stereoisomers (73mg, 25%), then 6β-(D-2-amino-3-phenylbut-3-enamido)penicillanic acid (35mg, 12%); $\nu_{max}$ (KBr) 1767, 1675, 1603, 1511, 1398 and 1318cm$^{-1}$; $\delta_H$(250MHz, $D_2O$) 1.42 (3H, s),1.51 (3H, s), 4.14 (1H, s), 4.72 (1H, s), 5.3-5.4 (2H, m), 5.46 and 5.55 (2H, 2s), and 7.41 (5H, s). [Mass spectrum: +ve ion (thioglycerol) $MH^+$ (376), $MNa^+$ (398)].

EXAMPLE 5

a. D,L-4,4-Dichloro-2-(2,2,2-trichloroethoxycarbonylamino)but-3-enoic acid

D,L-2-Amino-4,4-dichlorobut-3-enoic acid hydrochloride (2.07g, 10mmol) was treated with 2,2,2-trichloroethyl chloroformate (1.5ml, 11mmol) at pH9.25 ± 0.25, using the method described in example 2a, to provide the title compound (1.623g, 47%), mp 155-156°C from ethyl acetate-cyclohexane; $\delta_H$(60MHz, $(CD_3)_2CO$) 4.81 (2H, s), 5.16 (1H, dd, $J_{H-2,H-3}$ 9Hz, $J_{H-2,\ N-H}$ 8Hz), 6.22 (1H, d, $J_{H-3,H-2}$ 9Hz), 7.16 (1H, d, $J_{NH,\ H-2}$ 8Hz), and 9.65 (1H, br s).

b. Sodium 6β-[D,L-4,4-dichloro-2-(2,2,2-trichloroethoxycarbonylamino)but-3-enamido]penicillanate

Prepared using the method described in example 2b (52%); $\delta_H$(60MHz, $(CD_3)_2CO$) 1.53 (3H, s), 1.63 (3H, s), 4.27 (1H, s), 4.67 (2H, s), 4.7-5.2 (1H, m), 5.2-5.5 (2H, m), 6.00 (1H, d, J 9Hz), 7.0-7.7 (2H, m), and 8.85 (1H, br s).

c. 6β-(2-Amino-4,4-dichlorobut-3-enamido)penicillanic acids

Deprotection of sodium 6β-[D,L-4,4-dichloro-2-(2,2,2-trichloroethoxycarbonylamino)but-3-enamido] penicillinate (0.725g) as described in example 2c and chromatography on HP20SS eluting with 1% then 2% THF in water provided 6β-(L-2-amino-4,4-dichlorobut-3-enamido)penicillanic acid (55mg, 11%); $\nu_{max}$ (KBr) 1766, 1675, 1608, 1507, 1397 and 1320cm$^{-1}$; $\delta_H$(250MHz, $D_2O$) 1.50 (3H, s), 1.61 (3H, s), 4.24 (1H, s), 4.44 (1H, d, J 9Hz), 5.43 (1H, d, J 4Hz), 5.57 (1H, d, J 4Hz), and 6.07 (1H, d, J 9Hz); followed by a mixture of D and L stereoisomers (51mg, 11%); then 6β-(D-2-amino-4,4-dichlorobut-3-enamido)penicillanic acid (45mg, 9%); $\nu_{max}$ (KBr) 1770, 1690, 1615, 1520, 1396 and 1320cm$^{-1}$; $\delta_H$(250MHz, $D_2O$) 1.50 (3H, s) 1.60 (3H, s), 4.27 (1H, s), 4.98 (1H, d, J 10Hz), 5.46 (1H, d, J 4Hz), 5.56 (1H, d, J 4Hz), and 6.22 (1H, d, J 10Hz), [Mass spectrum: +ve ion (thioglycerol) $MH^+$ (368), $MNa^+$ (390)].

EXAMPLE 6

a. D-3-Methyl-2-(2,2,2-trichloroethoxycarbonylamino) but-3-enoic acid

D-2-Amino-3-methylbut-3-enoic acid (2.630g, 17.3mmol, J.E. Baldwin, S.B.Haber, C. Hoskins and L.I. Kruse, J. Org. Chem., 1977,42, 1239, S. Uyeo and H. Ona, Chem. Pharm. Bull., 1980,28, 1563) was converted to the title compound as described in example 2a (3.452g, 69%), m.p. 95-99°C from cyclohexane; $\delta_H$(60MHz, $(CD_3)_2CO$) 1.87 (3H, d, J1-2Hz), 4.82 (2H, s), 4.8-5.3 (3H, m), 7.0 (1H, br s), and 10.5 (1H, s).

b. Sodium 6,β-[D-3-methyl-2-(2,2,2-trichloroethoxy-carbonylamino)but-3-enamido]penicillanate

Prepared as described in example 2b (88%).

c. 6,β-(D-2-Amino-3-methylbut-3-enamido)penicillanic-acid

Deprotection of sodium 6,β-[D-3-methyl-2-(2,2,2-trichloroethoxycarbonylamino)but-3-enamido] penicillinate was performed as described in example 2c. Chromatography of the crude product on HP20SS, eluting with 0.66-1.25% THF in water, provided the title compound (50%); $\nu_{max}$ (KBr) 2971, 1771, 1686, 1599, 1520, 1389 and 1315cm$^{-1}$; $\delta_H$(250MHz, $D_2O$) 1.48 (3H, s), 1.60 (3H, s), 1.80 (3H, s), 4.22 (1H, s), 4.64

(1H, s), 5.33 and 5.34 (2H, 2s), 5.51 (1H, d, J 4Hz), and 5.54 (1H, d, J 4Hz). [Mass spectrum: +ve ion (thioglycerol) $MH^+$ (314), $2M+H^+$ (627)].

EXAMPLE 7

a. Sodium 7,β-[D-3-methyl-2-(2,2,2-trichloroethoxy carbonylamino)but-3-enamido]cephalosporanate

Prepared as described in example 2b, using 7-ACA instead of 6-APA (37%).

b. 7,β-(D-2-Amino-3-methylbut-3-enamido)cephalosporanic acid

Deprotection of sodium 7,β-[D-3-methyl-2-(2,2,2-trichloroethoxycarbonylamino)but-3-enamido] cephalosporanate was performed as described in example 2c. Chromatography of the crude product on HP20SS, eluting with 1¼-2½% THF in water, provided the title compound (33%); $\nu_{max}$ (KBr) 1770, 1693, 1602, 1387, 1340 and 1234cm$^{-1}$; $\delta_H$(250MHz, $D_2O$) 1.81 (3H, s), 3.35 and 3.63 (2H, 2d, J 18Hz), 4.64 (1H, s), 4.65-4.86 (2H, 2d, under HOD), 5.15 (1H, d, J 4.7Hz), 5.35 (2H, m), and 5.73 (1H, d, J 4.7Hz). [Mass spectrum: +ve ion (thioglycerol) $MH^+$-AcOH (310), $MH^+$ (370), $2M+H^+$ (739)].

EXAMPLE 8

a. Sodium 7,β-[D-3-methyl-2-(2,2,2-trichloroethoxy cabonylamino)but-3-enamido]-3-desacetoxycephalosporanate

Prepared as described in example 2b, using 7-ADCA instead of 6-APA (47%).

b. 7,β-[D-2-Amino-3-methylbut-3-enamido]-3-desacetoxy cephalosporanic acid

Deprotection of sodium 7,β-[D-3-methyl-2-(2,2,2-trichloroethoxycarbonylamino)but-3-enamido]-3-deacetoxycephalosporanate was performed as described in example 2c. Chromatography of the crude product on HP20SS, eluting with 1½-2% THF in water, provided the title compound (50%); $\nu_{max}$ (KBr) 3403, 2976, 1757, 1688, 1584 and 1359cm$^{-1}$; $\delta_H$(250MHz, $D_2O$) 1.80 (3H, s), 1.87 (3H, s), 3.18 and 3.56 (2H, 2d, J 18Hz), 4.57 (1H, s), 5.08 (1H, d, J 4.5Hz), 5.30 (2H, br s), and 5.62 (1H, d, J 4.5Hz). [Mass spectrum: +ve ion (thioglycerol) $MH^+$ (312), $2M+H^+$ (623), $3M+H^+$ (936)].

EXAMPLE 9

a. D,L-3-Ethyl-2-(2,2,2-trichloroethoxycarbonylamino) but-3-enoic acid

D,L-2-Amino-3-ethylbut-3-enoic acid hydrochloride (260mg, 1.57mmol, F. Heinzer and D. Bellus, Helv. Chim. Acta., 1981, 64, 2279) was converted to the title compound as described in example 2a, (320mg, 67%); $\delta_H$(60MHz, $(CD_3)_2CO$) 1.09 (3H, t, J 7Hz), 2.25 (2H, q, J 7Hz), 4.80 (2H, s), 4.85-5.3 (3H, m), 6.95 (1H, br d), and 9.82 (1H, s).

b. Sodium 6β-[D,L-3-ethyl-2-(2,2,2-trichloroethoxy carbonylamino)but-3-enamido]penicillanate

Prepared as described in example 2b (78%).

c. 6,β-(2-Amino-3-ethylbut-3-enamido)penicillanic acids

Deprotection of sodium 6β-[D,L-3-ethyl-2-(2,2,2-trichloroethoxycarbonylamino)but-3-enamido] penicillinate (417mg) was performed as described in example 2c. Chromatography of the crude product on

HP20SS, eluting with 0.75 -1% THF in water provided 6,β-(L-2-amino-3-ethylbut-3-enamido)penicillanic acid (95mg, 36%); $\nu_{max}$ (KBr) 2968, 1771, 1686, 1600, 1516, 1394 and 1316$cm^{-1}$; $\delta_H$(250MHz, $D_2O$) 1.06 (3H, t, J 7.3Hz), 1.50 (3H, s), 1.57 (3H, s), 2.08 (2H, m), 4.25 (1H, s), 4.68 (1H, s), 5.37 (1H, s), 5.40 (1H, s), 5.46 (1H, d, J 4.0Hz), and 5.58 (1H, d, J 4.0Hz). [Mass spectrum: +ve ion (thioglycerol) $MH^+$ (328)]; followed by a mixture of D and L stereoisomers (47mg, 18%); then 6,β-(D-2-amino-3-ethylbut-3-enamido)penicillanic acid (20mg, 7.6%); $\nu_{max}$ (KBr) 2968, 1767, 1688, 1597, 1395 and 1320$cm^{-1}$; $\delta_H$(250MHz, $D_2O$) 1.07 (3H, t, J 7.3Hz), 1.48 (3H, s), 1.60 (3H, s), 2.06 (2H, m), 4.21 (1H, s), 4.64 (1H, s), 5.35 (1H, s), 5.37 (1H, s), and 5.52 (2H, m). [Mass spectrum: +ve ion (thioglycerol) $MH^+$ (328), $MH^+$ + thioglycerol (436), $2M+H^+$ (655)]

Example 10

a. D,L-3-Methyl-2-(2,2,2-trichloroethoxycarbonyl-amino)pent-3(E)-enoic acid

D,L-2-Amino-3-methylpent-3(E)-enoic acid hydrochloride (1.36g, 8.2mmol) [for preparation see F. Heinzer and D. Bellus, Helv. Chim. Acta, 1981, 64, 2279] was converted to the title compound as described in Example 2a (1.62g, 65%), m.p. 133-137°C (chloroform/cyclohexane); (Found: C, 35.90; H, 4.09; N, 4.61; Cl 34.70%. $C_9H_{12}Cl_3NO_4$ requires C, 35.49; H, 3.97; N, 4.60; Cl, 34.92%); $\delta_H$(250MHz, $(CD_3)_2CO$) 1.64 (3H, d, J 6.8Hz), 1.71 (3H, s), 4.73 (1H, d, J 7.9Hz), 4.79 (2H, s), 5.68 (1H, q, J 6.5Hz), 7.12 (1H, br d), and 11.1 (1H, br s), some of the Z isomer of the title compound, E:Z ratio 8:1, was also present; $\delta_H$ inter alia 5.32 (d, J 7.8Hz). [Mass spectrum: +ve ion (ammonia) $MH^+$ (304), $MNH^+_4$ (321)].

b. 6β-[D,L-3-Methyl-2-(2,2,2-trichloroethoxy-carbonylamino)pent-3(E)-enamido]penicillanic acid

Prepared as described in Example 2b, but not converted to the sodium salt (79%).

c. 6β-(2-Amino-3-methylpent-3(E)-enamido)penicillanic acids

Deprotection of 6β-[D,L-3-methyl-2(2,2,2-trichloro-ethoxycarbonylamino)pent-3(E)-enamido]penicillanic acid (1.58g) was performed as described in Example 2c. Chromatography of the crude product on HP20SS, eluting with 0.5-0.8% THF in water, provided 6β-(L-2-amino-3-methylpent-3-(E)-enamido)penicillanic acid (179mg, 17%); $\nu_{max}$ (KBr) 2969, 1771, 1688, 1602, 1515, 1388 and 1316cm$^{-1}$; $\delta_H$(250MHz, $D_2O$) 1.49 (3H, s), 1.55 (3H, s),

1.64 (3H, s), 1.69 (3H, d, J 6.6Hz), 4.25 (1H, s) 4.62 (1H, s), 5.44 (1H, d, J 3.9Hz), 5.59 (1H, d, J 3.9Hz), and 5.95 (1H, br q, J 6.6Hz). [Mass spectrum: +ve ion (thioglycerol) $\underline{M}H^+$ (328)]; followed by a mixture of D- and L- stereoisomers (354mg, 34%); then 6β-(D-2-amino-3-methylpent-3(E)-enamido)penicillanic acid (111mg, 11%); $\nu_{max}$ (KBr) 2970, 1767, 1684, 1599, 1522, 1390, and 1318cm$^{-1}$; $\delta_H$(250MHz, $D_2O$) 1.49 (3H, s), 1.59 (3H, s), 1.65 (3H, s), 1.69 (3H, d, J 6.9Hz), 4.22 (1H, s), 4.56 (1H, s), 5.50 (1H, d, J 3.9Hz), 5.54 (1H, d, J 3.9Hz) and 5.91 (1H, br q). [Mass spectrum: +ve ion (thioglycerol) $\underline{M}H^+$ (328)].

Example 11

a. D,L-2-(2,2,2-Trichloroethoxycarbonylamino)pent-3(E)-enoic acid

D,L-2-Amino-pent-3(E)-enoic acid hydrochloride (1.97g crude) [for preparation see W.J. Greenlee, J.Org.Chem., 1984, 49, 2632] was converted to the title compound as described in Example 2a (0.534g), m.p. 139-140°C; (Found: C, 33.33; H, 3.38; N, 4.86; Cl, 36.17%. $C_8H_{10}Cl_3NO_4$ requires: C, 33.07; H, 3.47; N, 4.82; Cl 36.61%); $\delta_H$(250MHz, $(CD_3)_2CO$) 1.72 (3H, m), 4.77 (1H, m), 4.81 (2H, ABq, J 12.1Hz), 5.65 (1H, m), 5.91 (1H, m), and 7.19 (1H, d, J 7Hz); on irradiating at δ1.7 the signal at δ5.65 collapsed to a double doublet (J 15.3 and 6.9Hz) and the signal at δ5.91 collapsed to a double doublet (J 15.3 and 0.9Hz), confirming the E configuration.

b. 6β-[D,L-2-(2,2,2-Trichloroethoxycarbonylamino) pent-3(E)-enamido]penicillanic acid

Prepared as described in Example 2b, but not converted to the sodium salt (quantitative).

c. 6β-(2-Amino-pent-3(E)-enamido)penicillanic acids

Deprotection of 6β-[D,L-2-(2,2,2-trichloroethoxycarbonylamino)pent-3(E)-enamido]penicillanic acid (690mg, 1.7mmol) was performed as described in Example 2c. Chromatography of the crude product on HP20SS, eluting with 0.5-1% THF in water, provided 6β-(L-2-amino-pent-3(E)-enamido)penicillanic acid (105mg, 20%); $\nu_{max}$(KBr) 2971, 1766, 1683, 1596, 1520, 1395 and 1317cm$^{-1}$; $\delta_H$(250MHz, $D_2O$) 1.54 (3H, s), 1.63 (3H, s), 1.80 (3H, d, J 6.3Hz), 4.29 (1H, s), 4.63 (1H, d, J 8.3Hz), 5.5-5.7 (3H, m), and 6.19 (1H, m). [Mass spectrum: +ve ion (thioglycerol) $MH^+$ (314)]; followed by a mixture of D- and L- stereoisomers (106mg, 20%); then 6β-(D-2-aminopent-3(E)-enamido)penicillanic acid (129mg, 24%); $\nu_{max}$ (KBr) 3411, 2969, 1764, 1683, 1602, 1394 and 1318cm$^{-1}$; $\delta_H$(250MHz, $D_2O$) 1.54 (3H, s), 1.64 (3H, s), 1.81 (3H, dd, J 6.4 and 1.4Hz), 4.27 (1H, s), 4.63 (1H, d, J 8.5Hz), 5.5-5.7 (3H, m), and 6.18 (1H, m).

Example 12

a. Ethyl 2-methoxyimino-3-methyl-hex-3(E),5-dienoate

Allyl triphenylphosphonium bromide (9.582g, 25mmol) was suspended in dry ether (150ml) and treated slowly with n-butyllithium (1.6M, 15.6ml, 25mmol) at ca. 5°C. The mixture was stirred at room temperature for 2.5h, then ethyl 2-methoxyimino-3-oxo-butanoate (4.325g, 25mmol) in dry ether (100ml) was added slowly and the mixture stirred for 3h. After filtering, the solution was washed with saturated ammonium chloride (100ml) and brine (100ml), dried and evaporated to give a red oily solid (3.48g). Flash chromatography on silica gel, using 0-5% ethyl acetate in cyclohexane as eluant, gave

a colourless oil (1.24g, 25%); $\delta_H$(60MHz, $CDCl_3$) 1.32 (3H, t, J 7Hz), 1.96 (3H, s), 3.92 (3H, s), 4.38 (2H, q, J 7Hz), and 5.2-5.6 and 6.0-7.2 (4H, 2m).

b. Ethyl 2-amino-3-methyl-hex-3(E),5-dienoate

Ethyl 2-methoxyimino-3-methyl-hex-3(E),5-dienoate (1.23g, 6.2mmol) in ethanol (30ml), water (15ml) and formic acid (30ml) was cooled to *ca.* 5°C and treated portionwise with zinc dust (2.85g, 44mmol). The mixture was then stirred at room temperature for 1h, filtered and evaporated. The residue was dissolved in water, washed wih ether (2 x 30ml), adjusted to pH7 and extracted with ethyl acetate (3 x 40ml). The combined extracts were washed with brine, dried and evaporated to give the title compound as an orange oil (0.545g, 52%); $\delta_H$(60MHz, $CDCl_3$) 1.22 (3H, t, J 7Hz), 1.80 (3H, s), 2.80 (2H, s), 3.9-4.5 (3H, m + q, J 7Hz), and 5.0-5.5 and 6.0-7.0 (4H, 2m).

c. Ethyl 3-methyl-2-(2,2,2-trichloroethoxycarbonylamino)hex-3(E),5-dienoate

2,2,2-Trichlorethyl chloroformate (0.54ml, 3.9mmol) was added slowly to a stirred solution of ethyl 2-amino-3-methyl-hex-3(E),5-dienoate (0.545g, 3.22mmol) in THF (25ml) and water (25ml), maintained at pH 7.5. The mixture was stirred for 1.5h, then extracted with ethyl acetate (3 x 25ml). The combined extracts were washed with brine, dried and evaporated to give the crude product as a red oil. Flash chromatography on silica gel, using 0-10% ethyl acetate in hexane as eluant, gave the title compound as a yellow oil (0.745g, 67%); $\delta_H$(250MHz, $CDCl_3$) 1.29 (3H, t, J 7.1Hz), 1.80 (3H,

s), 4.24 (2H, m), 4.73 (2H, s), 4.78 (1H, d, J 7.4Hz), 5.20 (1H, d, J 10.2Hz), 5.29 (1H, d, J 16.8Hz), 5.91 (1H, br d), 6.18 (1H, d, J 10.8Hz), and 6.54 (1H, ddd, J 16.8, 10.8, 10.2Hz).

d. 3-Methyl-2-(2,2,2-trichloroethoxycarbonylamino) hex-3(E),5-dienoic acid

Ethyl 3-methyl-2-(2,2,2-trichloroethoxycarbonylamino) hex-3(E),5-dienoate (0.73g, 2.12mmol) in ethanol (10ml) was stirred with aqueous sodium hydroxide (1M, 2.3ml, 2.3mmol) for 1h. The solution was diluted with water, washed with ether (2 x 20ml), acidified and extracted with ethyl acetate (3 x 30ml). The combined extracts were washed with brine, dried and evaporated to yield the title compound as a pale yellow oil (0.530g, 79%); $\delta_H$(60MHz, $(CD_3)_2CO$) 1.9 (3H, s), 4.8 (2H, s), 4.85 (1H, d), 5.1-5.5 (2H, m), 6.1-7.2 (3H, m), and 9.25 (1H, s).

e. 6β-[D,L-3-Methyl-2-(2,2,2-trichloroethoxycarbonylamino)hex-3(E),5-dienamido]penicillanic acid

Prepared as described in Example 2b, but not converted to the sodium salt (71%).

f. 6β-(2-Amino-3-methylhex-3(E),5-dienamido) penicillanic acids

Deprotection of 6β-[D,L-3-methyl-2-(2,2,2-trichloroethoxycarbonylamino)hex-3(E),5-dienamido]penicillanic acid (0.585g, 1.14mmol) was performed as desribed in Example 2c. Chromatography of the crude product on HP20SS, eluting with 0.5-2.5% THF in water, provided 6β-(L-2-amino-3-methylhex-3(E),5-dienamido)penicillanic acid (70mg, 18%); $\nu_{max}$(KBr) 2970, 1771, 1688, 1598, 1515, 1393 and 1314$cm^{-1}$; $\delta_H$(250MHz, $D_2O$) 1.48 (3H,

s), 1.52 (3H, s), 1.78 (3H, s), 4.24 (1H, s), 4.68 (1H, s), 5.37 (1H, d, J 10.3Hz), 5.44 (1H, d, J 4Hz), 5.45 (1H, d, J 16.8Hz), 5.59 (1H, d, J 4Hz), 6.40 (1H, d, J 10.8Hz), and 6.64 (1H, ddd, J 16.8, 10.8, 10.3Hz). [Mass spectrum: +ve ion (thioglycerol) $\underline{M}H^+$ (340)]; followed by a mixture of D- and L- stereoisomers (100mg, 26%); then 6β-(D-2-amino-3-methylhex-3(E), 5-dienamido)penicillanic acid (58mg, 15%); $\nu_{max}$ (KBr) 3401, 2972, 1766, 1683, 1595, 1395 and 1318cm$^{-1}$; $\delta_H$(250MHz, $D_2O$) 1.46 (3H, s), 1.55 (3H, s), 1.81 (3H, d, J 0.6Hz), 4.21 (1H, s), 4.63 (1H, s), 5.36 (1H, d, J 10.2Hz), 5.40-5.48 (2H, m), 5.54 (1H, d, J 3.8Hz), 6.36 (1H, d, J 10.9Hz), and 6.65 (1H, ddd, J 16.8, 10.9, 10.2Hz). [Mass spectrum: +ve ion (thioglycerol) $\underline{M}H^+$ (340)].

Example 13

a. Ethyl 3-bromo-2-methoxyiminopropanoate

Ethyl bromopyruvate (12.6ml, 0.1mol) and methoxyamine hydrochloride (8.352g, 0.1mol) were dissolved in ethanol (120ml) and stirred for 2h at room temperature. After evaporation, the residue was taken up in ethyl acetate, washed with water and brine, dried and evaporated. Flash chromatography on silica gel, using 0-12% ethyl acetate in hexane as eluant, gave the title compound as a pale yellow oil (21.4g, 96%); (Found: $M^+$, 222.9843. $C_6H_{10}BrNO_3$ requires $\underline{M}$,222.9845); $\nu_{max}$ (film) 1720, 1370, 1330, 1200, 1180, 1050 and 1020cm$^{-1}$; $\delta_H$(250MHz, $CDCl_3$) 1.38 (3H, t, J 7.1Hz), 4.18 and 4.21 (together 3H, 2s, E and Z isomers), 4.17 and 4.36 (together 2H, 2s, E and Z isomers), and 4.38 (2H, q, J 7.1Hz).

b. <u>2-Ethoxycarbonyl-2-methoxyiminoethyltriphenyl-phosphonium bromide</u>

Triphenylphosphine (34.10g, 0.13mol) and ethyl 3-bromo-2-methoxyiminopropanoate (30.0g, 0.13mol) were dissolved in THF (220ml) and refluxed for 5h. After cooling, the product was filtered off, washed with ether and dried to give a white solid (46.51g, 74%), m.p. 137-139°C; $\nu_{max}$ (KBr) 1715, 1434, 1108, 1040, 742, 691 and 510cm$^{-1}$; $\delta_H$(250MHz, $CDCl_3$) 1.15 (3H, t, J 7.1Hz), 3.80 (3H, s), 4.12 (2H, q, J 7.1Hz), 5.25 (2H, d, J 16.3Hz), and 7.66-7.90 (15H, m). A second crop of the <u>title compound</u> was obtained, after a further 30h of refluxing, as a buff powder (9.77g, 15%), m.p. 123-130°C.

c. <u>Ethyl 2-methoxyiminohex-3(E)-enoate</u>

2-Ethoxycarbonyl-2-methoxyiminoethyltriphenyl-phosphonium bromide (10g, 20mmol) was dissolved in N,N'-dimethylpropylene urea (DMPU) (4ml) and THF (20ml), cooled to <u>ca</u>. 5°C and treated dropwise with n-butyllithium (1.6M, 12.8ml, 20mmol). The mixture was then stirred for 1.5h, cooled, and propanal (2ml, 28mmol) in THF (10ml) added slowly. Stirring was continued overnight, the mixture evaporated, the residue suspended in water and extracted with ether (2 x 60ml). The combined extracts were washed with brine, dried and evaporated. Flash chromatography, using 0-5% ethyl acetate in hexane as eluant, gave the <u>title compound</u> as a yellow oil, (1.85g, 50%); (Found: <u>M</u>$^+$, 185.1052. $C_9H_{15}NO_3$ requires <u>M</u>$^+$, 185.1052); $\nu_{max}$ (film) 2970, 2930, 1730, 1260, 1160, 1120 and 1050cm$^{-1}$; $\delta_H$(250MHz, $CDCl_3$) 1.07 (3H, t, J 7.4Hz), 1.37 (3H, t, J 7.1Hz), 2.24 (2H, m), 4.05 (3H, s), 4.34 (2H, q, J 7.1Hz), 6.50 (1H, dt, J 16.3, 1.5Hz), and 6.77 (1H, dt,

J 16.3, 6.4Hz); other signals corresponding to the Z olefin were present in the spectrum, and integration showed the product to be a 9:1 E:Z mixture.

d. Ethyl 2-aminohex-3(E)-enoate

Prepared as described in Example 12b (84%); $\nu_{max}$ (film) 2960, 2930, 1730, 1180, 1020, and 970cm$^{-1}$; $\delta_H$(60MHz, $CDCl_3$) 1.0 (3H, t, J 7.8Hz), 1.25 (3H, t, J 7.2Hz), 2.05 (2H, m), 2.55 (2H, s), 4.0-4.5 (3H, m+q, J 7.2Hz), and 5.35-6.2 (2H, m).

e. Ethyl 2-(2,2,2-trichloroethoxycarbonylamino)-hex-3(E)-enoate

Prepared as described in Example 12c (77%); $\delta_H$(60MHz, $CDCl_3$) 1.0 (3H, t, J 7.5Hz), 1.3 (3H, t, J 7.2Hz), 2.1 (2H, m), 4.25 (2H, q, J 7.2Hz), 4.75 (2H, s), 4.95 (1H, m), and 5.3-6.25 (3H, m).

f. 2-(2,2,2-Trichloroethoxycarbonylamino)hex-3(E)-enoic acid

Prepared as described in example 12d, using only one equivalent of base (68%), m.p. 120-121$^oC$ (ethyl acetate/cyclohexane); (Found: $M^+$-$H_2O$, 284.9733. $C_9H_{10}NO_3Cl_3$ requires M, 284.9726; found $M^+$-$CO_2H$, 257.9863. $C_8H_{11}NO_2Cl_3$ requires $M^+$, 257.9855); $\nu_{max}$ (KBr) 3307, 1736, 1710, 1696, 1534, 1249 and 721cm$^{-1}$; $\delta_H$(250MHz, $(CD_3)_2CO$) 0.99 (3H, t, J 7.5Hz), 2.1 (2H, m), 4.75-4.87 (3H, m), 5.64 (1H, ddt, J 15.4, 6.6, 1.5Hz), 5.95 (1H, dtd, J 15.4, 6.3, 1.2Hz), and 7.21 (1H, br d), (also contained a trace of the Z olefin).

g. 6β-[D,L-2-(2,2,2-Trichloroethoxycarbonylamino)-hex-3(E)-enamido]penicillanic acid

Prepared as described in Example 2b, but not converted to the sodium salt (91%).

h. 6β-(2-Aminohex-3(E)-enamido)penicillanic acids

Deprotection of 6β-[D,L-2(2,2,2-trichloroethoxycarbonylamino)hex-3(E)-enamido]penicillanic acid (0.89g, 1.8mmol) was performed as described in Example 2c. Chromatography of the crude product on HP20SS, eluting with 1-2% THF in water, provided 6β-(L-2-aminohex-3(E)-enamido)penicillanic acid (125mg, 21%); $\nu_{max}$ (KBr) 2967, 1771, 1686, 1602, 1515, 1393 and 1317cm$^{-1}$; $\delta_H$(250MHz, $D_2O$) 0.98 (3H, t, J 7.5Hz), 1.50 (3H, s), 1.59 (3H, s), 2.13 (2H, m), 4.25 (1H, s), 4.61 (1H, d, J 8.4Hz), 5.48 (1H, d, J 4.1Hz), 5.59 (1H, d, J 4.1Hz), 5.50-5.61 (1H, m), and 6.21 (1H, dt, J 15.4, 6.3Hz); followed by a mixture of D- and L- stereoisomers (63mg, 11%); then 6β-(D-2-aminohex-3(E)-enamido)penicillanic acid (80mg, 13%); $\nu_{max}$ (KBr) 2967, 1771, 1687, 1603, 1520, 1393 and 1318cm$^{-1}$; $\delta_H$(250MHz, $D_2O$) 1.00 (3H, t, J 7.5Hz), 1.49 (3H, s), 1.59 (3H, s), 2.13 (2H, m), 4.23 (1H, s), 4.60 (1H, d, J 8.6Hz), 5.49 (1H, d, J 3.9Hz), 5.55 (1H, d, J 3.9Hz), 5.52-5.60 (1H, m), and 6.20 (1H, dtd, J 15.2, 6.3, 0.5Hz). [Mass spectrum: +ve ion (thioglycerol) $\underline{M}H^+$ (328); -ve ion $\underline{M}^+$-H (326)].

Example 14

a. Ethyl 4-cyclohexyl-2-methoxyiminobut-3(E)-enoate

Prepared as described in Example 13c, using one equivalent (20mmol) of cyclohexane carboxaldehyde, to give, after chromatography, the title compound (2.60g, 54%); (Found: $\underline{M}^+$, 239.1523. $C_{13}H_{21}NO_3$ requires $\underline{M}$, 239.1521); $\delta_H$(250MHz, $CDCl_3$) 1.07-1.3 and 1.6-1.85 (10H, 2m), 1.36 (3H, t, J 7.1Hz), 2.10 (1H, m), 4.04 (3H, s), 4.34 (2H, q, J 7.1Hz), 6.47 (1H, dd, J 16.4, 1.0Hz), and 6.65 (1H, dd, J 16.4, 6.8Hz), (less than 5% of another isomer was present).

b. Ethyl 2-amino-4-cyclohexylbut-3(E)-enoate

Prepared as described in Example 12b (44%); $\delta_H$(60MHz, $CDCl_3$) 1.0-2.2 (14H, m+t, J 7Hz), 2.6 (2H, s), 4.05-4.5 (3H, m+d, J 7Hz), and 5.35-6.1 (2H, m).

c. Ethyl 4-cyclohexyl-2-(2,2,2-trichloroethoxycarbonylamino)but-3(E)-enoate

Prepared as described in example 12c (42%); $\delta_H$(60MHz, $CDCl_3$) 1.0-2.3 (14H, m+t, J 7Hz), 4.25 (2H, q, J 7Hz), 4.75-5.05 (3H, m+s) and 5.25-6.15 (3H, m).

d. 4-Cyclohexyl-2-(2,2,2-trichloroethoxycarbonylamino)but-3(E)-enoic acid

Prepared as described in Example 12d, using one equivalent of base (70%), m.p. 168-170°C (ethyl acetate/ cyclohexane); $\delta_H$(250MHz, $(CD_3)_2CO$) 1.0-1.4 and 1.6-1.8 (10H, 2m), 2.01 (m under $D_5$-acetone), 4.74-4.86 (3H, m), 5.61 (1H, ddd, J 15.6, 6.6, 1.1Hz), 5.87 (1H, ddd, J 15.6, 6.6, 1.1Hz), and 7.21 (1H, br d, J 7.9Hz). [Mass spectrum: +ve ion (3-nitrobenzylalcohol (3-NOBA)/$Na^+$), sodium salt $\underline{M}Na^+$ (402)].

e. 6β-[D,L-4-Cyclohexyl-2-(2,2,2-trichloroethoxycarbonylamino)but-3(E)-enamido]penicillanic acid

Prepared as described in Example 2b, but not converted to the sodium salt (88%).

f. 6β-(2-Amino-4-cyclohexylbut-3(E)-enamido) penicillanic acids

Deprotection of 6β[D,L-4-cyclohexyl-2-(2,2,2-trichloroethoxycarbonylamino)but-3(E)-enamido]penicillanic acid (0.628g, 1.1mmol) was performed as described in Example 2c. Chromatography of the crude product on HP20SS, eluting with 7-25% THF in water, provided 6β-(L-2-amino-4-cyclohexylbut-3-(E)-enamido)penicillanic acid (50mg, 12%); $\nu_{max}$ (KBr) 2923, 2851, 1759, 1688, 1609, 1511, 1389 and 1319cm$^{-1}$; $\delta_H$(250MHz, $D_2O$) 1.0-1.8 (16H, 2m+2s), 2.06 (1H, m), 4.25 (1H, s), 4.28 (1H, d, J 7.5Hz), 5.43-5.60 (3H, m+2d, J 4.0Hz), and 5.96 (1H, dd, J 16.0, 6.6Hz); followed by a mixture of D- and L-stereoisomers (23mg, 5%); then 6β-(D-2-amino-4-cyclohexylbut-3(E)-enamido)penicillanic acid (80mg, 19%); $\nu_{max}$(KBr) 2924, 2850, 1771, 1683, 1599, 1515, 1393 and 1315cm$^{-1}$; $\delta_H$(250MHz, $D_2O$) 1.0-1.8 (16H, 2m+2s), 2.05 (1H, m), 4.22 (1H, s), 4.46 (1H, d, J 8.2Hz), 5.43-5.61 (3H, m+2d, J 3.9Hz), and 6.05 (1H, dd, J 15.6, 6.5Hz). [Mass spectrum: +ve ion (thioglycerol) $\underline{M}H^+$ (382)].

**Example 15**

a. Ethyl 2-methoxyimino-4-phenylbut-3(E)-enoate

Prepared as described in Example 13c, using one equivalent of benzaldehyde, to give, after chromatography, the title compound (77%); (Found: $M^+$, 233.1044. $C_{13}H_{15}NO_3$ requires $\underline{M}$, 233.1052); $\delta_H$(400MHz, $CDCl_3$) 1.40 (3H, t, J 7.1Hz), 4.13 (3H, s), 4.40 (2H, d, J 7.1Hz), 7.22 (1H, d, J 16.7Hz), 7.30-7.39 (3H, m), 7.51-7.53 (2H, m) and 7.59 (1H, d, J 16.7Hz) (single isomer).

b. 2-Methoxyimino-4-phenylbut-3(E)-enoic acid

Ethyl 2-methoxyimino-4-phenylbut-3(E)-enoate (2.91g, 12.5mmol) in ethanol (60ml) was treated with aqueous sodium hydroxide (1M, 37ml, 37mmol), at room temperature, for 1h. The solution was reduced in volume, diluted with water and washed with ether (2 x 50ml). The aqueous phase was then acidified to

pH2 and extracted with ethyl acetate (3 x 60ml). The combined extracts were washed with brine, dried and evaporated to give a white solid (2.42g), which was recrystallised from ethyl acetate/cyclohexane to give colourless needles (2.115g, 82%), m.p. 110-111$^{o}$C; (Found: C,64.67; H,5.36; N,6.68%. $C_{11}H_{11}NO_3$ requires: C,64.38; H,5.40; N, 6.83%); $\delta_H$(60MHz, $(CD_3)_2CO$) 4.1 (3H, s), 7.25 (1H, d, J 17Hz), 7.35-7.70 (5H, m), 7.85 (1H, d, J 17Hz), and 10.3 (1H, br s).

c. 2-Amino-4-phenylbut-3(E)-enoic acid

2-Methoxyimino-4-phenylbut-3(E)-enoic acid (0.595g, 2.9mmol) was dissolved in ethanol (15ml), water (7.5ml) and formic acid (15ml), cooled to *ca.* 5$^{o}$C, and zinc powder (1.0g, 15mmol) added portionwise. After five minutes the mixture was filtered and reduced in volume. The solid precipitate was filtered off, washed with acetone then ether, and dried *in vacuo* to give the *title compound* as a white crystalline solid (0.450g, 88%), m.p. 197-199$^{o}$C; $\nu_{max}$ (KBr) 2946, 1646, 1585, 1396, 1354, 1331, 962 and 734 cm$^{-1}$; $\delta_H$(250MHz, $D_2O$/DCl) 4.53 (1H, dd, J 8.5, 0.4Hz), 6.00 (1H, dd, J 15.9, 8.4Hz), 6.65 (1H, dd, J 15.9, 0.4Hz), and 7.05-7.23 (5H, m). [Mass spectrum: +ve ion (ammmonia) $MH^+$ (178)]. The aqueous filtrate afforded a second crop of the *title compound*, after HP20SS chromatography, (37mg, 7%).

d. 4-Phenyl-2-(2,2,2-trichloroethoxycarbonylamino)-but-3(E)-enoic acid

2-Amino-4-phenylbut-3(E)-enoic acid (0.46g, 2.5mmol) was suspended in water (15ml) and THF (15ml) and treated dropwise with 2,2,2-trichloroethyl chloroformate (0.5ml, 3.6mmol) in THF (5ml), while

maintaining the pH at 7.7± 0.2. The mixture was stirred for one hour, reduced in volume, washed with ether (2 x 30ml), acidified and extracted with ethyl acetate (3 x 30ml). The combined extracts were washed with brine, dried and evaporated. Recrystallisation, from ethyl acetate/cyclohexane, gave the title compound as a buff powder (295mg, 40%), m.p. 205-207°C; $\delta_H$(250MHz, $(CD_3)_2CO$) 4.85 and 4.88 (2H, ABq, J 12.2Hz), 5.03 (1H, m), 6.46 (1H, dd, J 16.0, 6.7Hz), 6.84 (1H, d, J 16.0Hz), and 7.25-7.50 (6H, m). [Mass spectrum: $M^+$ (351), $M^+-CO_2$ (306)].

e. 6β-[D,L-4-Phenyl-2-(2,2,2-trichloroethoxycarbonylamino)but-3(E)-enamido]penicillanic acid

Prepared as described in Example 2b, but not converted to the sodium salt (82%).

f. 6β-(2-Amino-4-phenylbut-3(E)-enamido)penicillanic acids

Deprotection of 6β-[D,L-4-phenyl-2-(2,2,2-trichloroethoxycarbonylamino)but-3(E)-enamido]penicillanic acid (1.4g, 2.5mmol) was performed as described in Example 2c. Chromatography of the crude product on HP20SS, eluting with 5-25% THF in water, provided 6β-(L-2-amino-4-phenylbut-3(E)-enamido)penicillanic acid (46mg, 5%); $\nu_{max}$ (KBr) 2970, 1773, 1690, 1602, 1517, 1395 and 1315cm$^{-1}$, $\delta_H$(250MHz, $D_2O$) 1.45 (6H, s), 4.22 (1H, s), 4.8 (1H obscured by HOD), 5.49 (1H, d, J 4.0Hz), 5.60 (1H, d, J 4.0Hz), 6.31 (1H, dd, J 15.9, 8.5Hz), 7.01 (1H, d, J 15.9Hz), and 7.36-7.58 (5H, m); followed by a mixture of D- and L-stereoisomers (106mg, 11%); then 6β-(D-2-amino-4-phenylbut-3(E)enamido)penicillanic acid (80mg, 9%); $\nu_{max}$(KBr) 2971, 1772, 1689, 1599, 1517, 1395 and

1317cm$^{-1}$; $\delta_H$(250MHz, $D_2O$) 1.40 (6H, s), 4.19 (1H, s), 4.8 (1H obscured by HOD), 5.45 (1H, d, J 3.7Hz). 5.55 (1H, d, J 3.7Hz), 6.29 (1H, dd, J 15.9, 8.6Hz), 7.0 (1H, d, J 15.9Hz), and 7.40-7.57 (5H, m). [Mass spectrum: +ve ion (thioglycerol) $\underline{M}H^+$ (376)].

Example 16

a. Ethyl 4-(4-chlorophenyl)-2-methoxyiminobut-3(E)-enoate

Prepared as described in Example 13c, using 0.97 equivalents (29mmol) of 4-chlorobenzaldehyde, to give, after chromatography, the title compound (3.31g, 43%); $\delta_H$(60MHz, $CDCl_3$) 1.4 (3H, t, J 7Hz), 4.1 (3H, s), 4.4 (2H, q, J 7Hz), 7.1 (1H, d, J 17Hz), 7.1-7.4 (4H, m), and 7.6 (1H, d, J 17Hz). [Mass spectrum: $\underline{M}^+$(269)].

b. 4-(4-Chlorophenyl)-2-methoxyiminobut-3(E)-enoic acid

Prepared as described in Example 15b (1.735g, 78%), m.p. 73-80°C (cyclohexane); $\delta_H$(60MHz, $(CD_3)_2CO$) 4.1 (3H, s), 7.1 (1H, d, J 17Hz), 7.5 (4H, m), 7.8 (1H, d, J 17Hz), and 10.7 (1H, br s).

c. 2-Amino-4-(4-chlorophenyl)but-3(E)-enoic acid

Prepared as described in Example 15c (1.213g, 80%); $\nu_{max}$ (KBr) 2950, 1643, 1586, 1490, 1392, 964 and 817cm$^{-1}$; $\delta_H$(250MHz, $D_2O$ + DCl) 4.38 (1H, dd, J 8.4, 0.6Hz), 5.82 (1H, dd, J 15.8, 8.4Hz), 6.45 (1H, d, J 15.8Hz), and 6.87-7.00 (4H, m).

d. 4-(4-Chlorophenyl)-2-(2,2,2-trichloroethoxycarbonylamino)but-3(E)-enoic acid

Prepared as described in Example 15d (0.784g, 36%), m.p. 188-193°C (ethyl acetate/cyclohexane); $\nu_{max}$ (KBr) 1728, 1510, 1491, 1406, 1246, 1093 and 809cm$^{-1}$;

$\delta_H$(250MHz, $(CD_3)_2CO$) 4.83 and 4.86 (2H, ABq, J 12.2Hz), 5.03 (1H, m), 6.49 (1H, dd, J 16.0, 6.6Hz), 6.84 (1H, dd, J 16.0, 0.9Hz) and 7.36-7.54 (5H, m). [Mass spectrum: M$^+$ (385), M$^+$-$CO_2H$ (340)].

e. 6β-[D,L-4-(4-chlorophenyl)-2-(2,2,2-trichloroethoxycarbonylamino)but-3(E)-enamido]penicillanic acid

Prepared as described in Example 2b, but not converted to the sodium salt (79%).

f. 6β-[D,L-2-Amino-4-(4-chlorophenyl)but-3(E)-enamido]penicillanic acid

Deprotection of 6β-[D,L-4-(4-chlorophenyl)-2-(2,2,2-trichloroethoxycarbonylamino)but-3(E)-enamido]penicillanic acid (0.89g, 1.5mmol) was performed as described in Example 2c. Chromatography of the crude product on HP20SS, eluting with 10-35% THF in water, provided the title compound (158mg, 27%); $\nu_{max}$ (KBr) 1773, 1688, 1594, 1513, 1491, 1392 and 1317cm$^{-1}$; $\delta_H$(250MHz, $D_2O$) 1.37 (3H, s), 1.43 (3H, s), 4.17 and 4.20 (together 1H, 2s), 4.8 (1H obscured by HOD), 5.42 and 5.47 (together 1H, 2d, J 3.7, 4.1Hz) ,5.53 and 5.58 (together 1H, 2d, J 3.7, 4.1Hz), 6.2-6.35 (together 1H, 2dd, J 15.9, 8.6Hz), 6.94 and 6.96 (together 1H, 2d, J 15.9Hz), and 7.36-7.50 (4H, m). [Mass spectrum: +ve ion (thioglycerol) MH$^+$ (410)].

## Example 17

a. Ethyl 2-methoxyimino-4-trifluoromethylbut-3-enoate

Methyltriphenylphosphonium bromide (7.145g, 20mmol) was suspended in THF (100ml), cooled to ca. -25$^o$C, and n-butyllithium (1.5M, 13.3ml, 20mmol) added slowly.

The mixture was stirred at -20°C for 1.5h, cooled to ca. -70°C and ethyl 2-methoxyimino-3-oxo-4,4,4-trifluorobutanoate (4.543g, 20mmol) [for preparation see C. Scolastico et.al., Synthesis, 1985, 850] in THF (20ml) added slowly. The mixture was then stirred while warming slowly to room temperature and overnight at room temperature. The mixture was then evaporated, the residue dissolved in ethyl acetate, washed with water and brine, dried and evaporated. The crude product was purified by flash chromatography on silica gel, using 0-5% ethyl acetate in hexane as eluant, to give the title compound as a volatile, colourless oil (1.13g, 25%); $\delta_H$(60MHz, $CDCl_3$) 1.35 (3H, t, J 7Hz), 4.05 (3H, s), 4.4 (2H, q, J 7Hz), and 5.85 and 6.25 (2H, 2m).

b. Ethyl 2-amino-3-trifluoromethylbut-3-enoate

Prepared as described in Example 12b (0.70g, 71%); $\delta_H$(60MHz, $CDCl_3$) 1.25 (3H, t, J 7Hz), 2.0 (2H, br s), 4.25 (3H, m+q, J 7Hz), and 5.85 and 6.0 (2H, 2m).

c. Ethyl 2-(2,2,2-trichloroethoxycarbonylamino)-3-trifluoromethylbut-3-enoate

Prepared as described in Example 12c (1.3g, quantitative); $\delta_H$(60MHz, $CDCl_3$) 1.3 (3H, t, J 7Hz), 4.35 (2H, q, J 7Hz), 4.8 (2H, s), 5.25 (1H, d, J 8Hz) 5.95 and 6.15 (2H, 2m), and 6.35 (1H, br d, J 8Hz). [Mass spectrum: $M^+$ (371)].

d. 2-(2,2,2-Trichloroethoxycarbonylamino)-3-trifluoromethylbut-3-enoic acid

Prepared as described in Example 12d (0.965g, 80%); $\delta_H$(60MHz, $(CD_3)_2CO$) 4.85 (2H, s), 5.15 (1H, d, J 8Hz), 6-6.3 (2H, m) and 7.4 (1H, br d, J 8Hz).

e. 6,β-[D,L-2-(2,2,2-trichloroethoxycarbonylamino)-3-trifluoromethylbut-3-enamido]penicillanic acid

Prepared as described in Example 2b, but not converted to the sodium salt (1.32g, 87%).

f. 6,β-(2-Amino-3-trifluoromethylbut-3-enamido)-penicillanic acids

Deprotection of 6β-[D,L-2-(2,2,2-trichloroethoxycarbonylamino)-3-trifluoromethylbut-3-enamido] penicillanic acid (1.32g, 2.4mmol) was performed as described in Example 2c. Chromatography of the crude product on HP20SS, eluting with 0.5-3% THF in water, provided 6β-(L-2-amino-3-trifluoromethylbut-3-enamido) penicillanic acid (80mg, 9%); $\nu_{max}$ (KBr) 1774, 1694, 1607, 1516, 1314, 1174 and 1130cm$^{-1}$; $\delta_H$(250MHz, $D_2O$) 1.50 (3H, s), 1.59 (3H, s), 4.25 (1H, s), 4.99 (1H, s), 5.50 (1H, d, J 4.0Hz), 5.59 (1H, d, J 4.0Hz), and 6.19 and 6.41 (2H, 2 br s); followed by a mixture of D- and L- stereoisomers (72mg, 8%); then 6,β-(D-2-amino-3-trifluoromethylbut-3-enamido)penicillanic acid (60mg, 7%); $\nu_{max}$ (KBr) 1772, 1694, 1520, 1318, 1246, 1176 and 1129cm$^{-1}$; $\delta_H$(250MHz, $D_2O$) 1.49 (3H, s), 1.60 (3H, s), 4.22 (1H, s), 4.96 (1H, s), 5.55 (1H, d, J 3.9Hz), 5.58 (1H, d, J 3.9Hz), and 6.21 and 6.42 (2H, 2 br s). [Mass spectrum: +ve ion (thioglycerol) $\underline{M}H^+$ (368)].

## Example 18

a. Methyl 5-chloromethyl-5-thien-2-yl-2-oxazolin-4-ylcarboxylate

Cuprous oxide (0.05g) was added to 2-chloroacetylthiophene (1.61g, 10mmol) and methyl isocyanoacetate (0.91ml, 10mmol) in toluene (25ml) then the mixture was stirred and heated at 70°C for 5h. The reaction mixture was concentrated *in vacuo* and the residue flash chromatographed on silica gel to give the *title*

compound (1.49g, 57%); (Found: $M^+$, 259.0067. $C_{10}H_{10}ClNO_3S$ requires M, 259.0069); $\delta_H$ (60MHz, $CDCl_3$) 3.33 (3H, s), 4.00 (2H, s), 5.02 (1H, d, J 2Hz), and 6.8-7.4 (4H, m).

b. Methyl 2-formamido-3-thien-2-ylbut-3-enoate

Activated zinc dust (2.3g) was added to methyl 5-chloromethyl-5-thien-2-yl-2-oxazolin-4-ylcarboxylate (4.59g) in THF (25ml) under argon and heated under reflux for 2h. Ethyl acetate (50ml) and 0.5N hydrochloric acid (50ml) were added, the mixture filtered and the filtrate extracted twice more with ethyl acetate. The extracts were washed with brine (3 x 50ml), dried, the solvent evaporated and the residue flash chromatographed on silica gel to give the title compound (3.01g, 61%); (Found: $M^+$, 225.0460. $C_{10}H_{11}NO_3S$ requires M, 225.0460); $\delta_H$(60MHz, $CDCl_3$) 3.78 (3H, s), 5.28 (1H, s), 5.62 (1H, s), 5.65 (1H, d, J 8Hz), 6.74 (1H, br d, J 8Hz, exch $D_2O$), 6.9-7.3 (3H, m), and 8.25 (1H, s).

c. Methyl 2-amino-3-thien-2-ylbut-3-enoate hydrochloride

Methanol was added to methyl 2-formamido-3-thien-3-ylbut-3-enoate (1.04g) in 5N hydrochloric acid (10ml) to give a homogeneous solution which was stirred overnight then evaporated *in vacuo* to give the title compound as a crude foam (0.99g, 92%); $\delta_H$ (60MHz, $CD_3OD$) 3.83 (3H, s), 5.23 (1H, s), 5.55 (1H, s), 5.83 (1H, s), and 6.9-7.5 (3H, m).

d. Methyl 3-thien-2-yl-2-(2,2,2-trichloroethoxycarbonylamino)but-3-enoate

Methyl 2-amino-3-thien-2-ylbut-3-enoate hydrochloride (0.99g, 4.2mmole) in 50% aqueous THF (40ml) was maintained at pH7± 0.5 with 2.5N sodium hydroxide

solution while 2,2,2-trichloroethyl chloroformate (0.725ml, 5.25mmol) was added dropwise. The solution was stirred for a further 0.5h at pH 7± 0.5 then extracted with chloroform (3 x 25ml). The extracts were washed with water (2 x 50ml), dried, the solvent evaporated in vacuo and the residue flash chromatographed on silica gel to provide the title compound (0.95g, 61%); (Found: $M^+$, 370.9557. $C_{12}H_{12}Cl_3NO_4S$ requires M, 370.9553); $\delta_H$ (60MHz, $CDCl_3$) 3.77 (3H, s), 4.73 (2H, s), 5.28 (1H, s), 5.33 (1H, d, J 8Hz) 5.63 (1H, s), 6.18 (1H, br d, J 8Hz, exch $D_2O$), and 6.85-7.3 (3H, m).

e. 3-Thien-2-yl-2-(2,2,2-trichloroethoxycarbonylamino)but-3-enoic acid

Methyl 3-thien-2-yl-2-(2,2,2-trichloroethoxycarbonylamino)but-3-enoate (1.54g 4.1mmol) in N sodium hydroxide (5ml) and methanol (5ml) was stirred for 2h. The solution was washed with chloroform (2 x 10ml), acidified to pH2.5 and extracted with chloroform (3 x 15ml). The extracts were washed with water (25ml), dried, the solvent evaporated in vacuo and the residue flash chromatographed on silica gel to give the title compound (1.04g, 70%); $\delta_H$ (60MHz,$(CD_3)_2CO$) 4.83 (2H, s), 5.37 (1H, d, J 8Hz), 5.38 (1H, s), 5.65 (1H, s), 6.9-7.5 (4H, m), and 8.47 (1H, br s, exch $D_2O$).

f. 6β-[D,L-3-Thien-2-yl-2-(2,2,2-trichloroethoxycarbonylamino)but-3-enamido]penicillanic acid

Prepared as described in Example 2b but the title compound isolated as the crude free acid (82%).

g. 6β-(2-Amino-3-thien-2-ylbut-3-enamido)penicillanic acids

6β-[D,L-3-Thien-2-yl-2-(2,2,2-trichloroethoxycarbonylamino)but-3-enamido]penicillanic acid (1.14g) was

deprotected as described in Example 2c. The crude product was chromatographed on HP20SS in 3% THF-water to give 6β-(L-2-amino-3-thien-2-ylbut-3-enamido) penicillanic acid (15mg); $\nu_{max}$ (KBr) 3349, 1766, 1669, 1601 and 1515cm$^{-1}$; $\delta_H$(250MHz, $D_2O$) 1.42 (3H, s), 1.46 (3H, s), 4.19 (1H, s), 5.09 (1H, s), 5.46 (1H, d, J 4.1Hz), 5.52 (1H, s), 5.58 (1H, d, J 4.1Hz), 5.87 (1H, s), and 7.0-7.5 (3H, m); followed by a mixture of D and L stereoisomers (15mg) then 6β-(D-2-amino-3-thien-2-yl-but-3-enamido)penicillanic acid (16mg); $\nu_{max}$ (KBr) 3373, 1766, 1675, 1599 and 1516cm$^{-1}$; $\delta_H$ (250MHz, $D_2O$) 1.44 (3H, s), 1.52 (3H, s), 4.17 (1H, s), 4.97 (1H, s), 5.47 (1H, d, J 3.9Hz), 5.48 (1H, s), 5.53 (1H, d, J 3.9Hz), 5.82 (1H, s), and 7.05-7.45 (3H, m). [Mass spectrum: +ve ion (thioglycerol) $\underline{M}H^+$ (382), $\underline{M}Na^+$ (404)].

## Example 19

a. 2-(Dichloroacetyl)thiophene

Sulphuryl chloride (21ml) was added dropwise to 2-acetylthiophene (16.2ml) in carbon tetrachloride (50ml) cooled in a water bath. The mixture was stirred for 1.5h then distilled to provide the title compound (19.19g), b.p. 144-148°C/12mm; $\delta_H$(60MHz, $CDCl_3$) 6.50 (1H, s), and 7.1-8.1 (3H, m); containing *ca.* 30% 2-(chloroacetyl)thiophene, $\delta_H$ (*inter alia*) 4.60 (2H, s).

b. Methyl 5-dichloromethyl-5-thien-2-yl-2-oxazolin-4-ylcarboxylate

2-(Dichloroacetyl)thiophene (6.77g, 34.5mmol, mixed with 2-(chloroacetyl)thiophene 2.37g) was added dropwise to methyl isocyanoacetate (2.3ml, 30mmol) and triethylamine (4.3ml, 30mmol) in toluene (30ml) cooled in an ice bath. The reaction was stirred for 24h at

room temperatu: then acetic acid (1.8ml) added followed by ethyl acetate (100ml). The mixture was washed with water (2 x 50ml) and brine (50ml), dried, concentrated and flash chromatographed on silica gel to give the title compound (7.30g, 83%); (Found: $M^+$, 292.9668. $C_{10}H_9Cl_2NO_3S$ requires M, 292.9680); $\delta_H$(250MHz, $CDCl_3$) 3.43 (3H, s), 5.13 and 5.26 (together 1H, 2d, J 2Hz, isomer ratio 1:4), 6.00 and 6.46 (together 1H, 2s, ratio 4:1), and 7.0-7.4 (4H, m).

c. Methyl 4-chloro-2-formamido-3-thien-2-ylbut-3-enoate

Methyl 5-dichloromethyl-5-thien-2-yl-2-oxazolin-4-yl-carboxylate was treated with activated zinc dust in THF as described in Example 18b. The crude product was chromatographed on silica gel to give the E-isomer of the title compound (9%). A sample was crystallised from ethyl acetate-hexane, m.p. 92-93°C; (Found: C, 46.29; H, 3.89; N, 5.43; Cl, 13.71, S, 12.18%. $C_{10}H_{10}ClNO_3S$ requires C, 46.25; H, 3.88; N, 5.39; Cl, 13.65; S, 12.35%); $\delta_H$ (60MHz, $CDCl_3$), 3.80 (3H, s), 6.11 (1H, d, J 8Hz), 6.62 (1H, s), 6.87 (1H, br d, J 8Hz, exch $D_2O$), 6.9-7.4 (3H, m), and 8.23 (1H, s).

Further elution of the column and crystallisation from ethyl acetate - hexane gave the Z-isomer of the title compound (42%), m.p. 117-9°C; (Found: C, 46.35; H, 3.87; N, 5.36; Cl, 13.47; S, 12.41%. $C_{10}H_{10}ClNO_3S$ requires C, 46.25; H, 3.88; N, 5.39; Cl, 13.65; S, 12.35%); $\delta_H$ (250MHz, $CDCl_3$) 3.79 (3H, s), 5.59 (1H, d, J 7.7Hz), 6.56 (1H, br d, J 7.7Hz), 6.61 (1H, s), 7.05-7.45 (3H, m), and 8.23 (1H, s). Confirmed as Z-isomer by N.O.E.

d. Methyl 2-amino-4-chloro-3-thien-2-ylbut-3(Z)enoate hydrochloride

Methyl 4-chloro-2-formamido-3-thien-2-ylbut-3(Z)-enoate (0.97g) was hydrolysed as described in Example 18c. The title compound was obtained as a crystalline solid by trituration of the crude product with ether (0.80g), m.p. 190-1°C; (Found: C, 40.03; H, 4.14; N, 5.25; S, 12.21%. $C_9H_{11}Cl_2NO_2S$ requires C, 40.31; H, 4.13; N, 5.22; S, 11.96%); $\delta_H$(60MHz, $CD_3OD$) 3.90 (3H, s), 5.33 (1H, s), 7.07 (1H, s), and 7.1-7.8 (3H, m).

e. Methyl 4-chloro-3-thien-2-yl-2(2,2,2-trichloroethoxycarbonylamino)but-3(Z)-enoate

Methyl 2-amino-4-chloro-3-thien-2-ylbut-3(Z)-enoate hydrochloride (1.22g) was acylated with 2,2,2-trichloroethyl chloroformate (0.665ml) as described in Example 18d. The title compound was crystallised from hexane (1.42g, 77%), m.p. 66-7°C; (Found: C, 35.55; H, 2.66; N, 3.39; Cl, 34.79; S, 7.82%. $C_{12}H_{11}Cl_4NO_4S$ requires C, 35.40; H, 2.72; N, 3.44; Cl, 34.83; S, 7.88%); $\delta_H$(60MHz, $CDCl_3$) 3.77 (3H, s), 4.73 (2H, s), 5.25 (1H, d, J 8Hz), 6.07 (1H, br d, J 8Hz, exch $D_2O$), 6.58 (1H, s), and 6.95-7.5 (3H, m).

f. 4-Chloro-3-thien-2-yl-2-(2,2,2-trichloroethoxycarbonylamino)but-3-(Z)-enoic acid

Methyl 4-chloro-3-thien-2-yl-2-(2,2,2-trichloroethoxycarbonylamino)but-3(Z)-enoate (1.42g) was hydrolysed as described in Example 18e. The title compound was crystallised from ethyl acetate-hexane (0.51g, 37%), m.p. 112-3°C; (Found; $\underline{M}^+$, 390.9009. $C_{11}H_9Cl_4NO_4S$ requires $\underline{M}$, 390.9007); $\delta_H$(60MHz, $(CD_3)_2CO$) 4.77 (3H, s), 5.38 (1H, d, J 8Hz), 6.70 (1H, s), 6.9-7.5 (4H, m), and 9.80 (1H, br s, exch $D_2O$).

g. 6β-[D,L-4-Chloro-3-thien-2-yl-2-(2,2,2-trichloroethoxycarbonylamino)but-3(Z)-enamido]penicillanic acid

Prepared as described in Example 2b but the title compound was isolated as the crude free acid (77%).

h. 6β-(D,L-2-Amino-4-chloro-3-thien-2-ylbut-3(Z)-enamido)penicillanic acid

6β-[D,L-4-Chloro-3-thien-2yl-2-(2,2,2-trichloroethoxycarbonylamino)but-3(Z)-enamido]penicillanic acid was deprotected as described in example 2c. The title compound was isolated as a white freeze dried solid after chromatography on HP20SS (7%); $\nu_{max}$(KBr) 3379, 1771, 1688, 1601, 1515 and 708cm$^{-1}$; $\delta_H$(250MHz, $D_2O$) 1.45, 1.47 and 1.56 (together 6H, 3s), 4.20 and 4.22 (together 1H, 2s), 5.18 and 5.20 (together 1H,2s), 5.45 and 5.54 (together 1H, 2d, J 4Hz), 5.59 and 5.60 (together 1H, 2d, J 4Hz), 7.00 and 7.02 (together 1H, 2s), and 7.1-7.2 (3H, m). [Mass spectrum: +ve ion (glycerol/DMSO) $\underline{M}H^+$ (416), 2$\underline{M}$+$H^+$ (831)].

## Example 20

a. Methyl 2-amino-4-chloro-3-thien-2-ylbut-3(E)-enoate hydrochloride

Methyl 4-chloro-2-formamido-3-thien-2-ylbut-3(E)-enoate (0.50g, from Example 19c) was hydrolyed as described in Example 18c. The title compound was obtained as a crude foam (0.50g, 96%); $\delta_H$(60MHz, $CD_3OD$) 3.85 (3H, s), 5.57 (1H, s), 6.97 (1H, s), and 7.0-7.6 (3H, m).

b. Methyl 4-chloro-3-thien-2-yl-2-(2,2,2-trichloroethoxycarbonylamino)but-3(E)-enoate

Methyl 2-amino-4-chloro-3-thien-2-ylbut-3(E)-enoate hydrochloride (0.50g) was acylated as described in Example 18d. The title compound was crystallised from hexane (0.63g, 84%), m.p. 67-9°C; (Found: C,35.64; H,

2.62; N, 3.28; Cl, 34.86; S, 7.93%. $C_{12}H_{11}Cl_4NO_4S$ requires C, 35.40; H, 2.72; N, 3.44; Cl, 34.83; S, 7.88%); $\delta_H$ (60MHz, $CDCl_3$) 3.78 (3H, s), 4.73 (2H, s), 5.83 (1H, d, J 8Hz), 6.15 (1H, d, J 8Hz), 6.60 (1H, s), and 6.85-7.3 (3H, m).

c. <u>4-Chloro-3-thien-2-yl-2-(2,2,2-trichloroethoxycarbonylamino)but-3(E)-enoic acid</u>

Methyl 2-amino-3-thien-2-yl-2-(2,2,2-trichloroethoxycarbonylamino)but-3(E)-enoate (0.52g), was hydrolysed as described in Example 18e to give the <u>title compound</u> as a foam (0.45g, 86%); $\delta_H$(60MHz, $(CD_3)_2CO$) 4.77 (2H, s), 5.88 (1H, d, J 7Hz), 6.78 (1H, s), 6.9-7.5 (4H, m), and 9.20 (1H, br s, exch $D_2O$).

d. <u>6β-[D,L-4-Chloro-3-thien-2-yl-2-(2,2,2-trichloroethoxycarbonylamino)but-3(E)-enamido]penicillanic acid</u>

Prepared as described in Example 2b to give the <u>title compound</u> as the free acid (quantitative).

e. <u>6B-(D,L-2-Amino-4-chloro-3-thien-2-ylbut-3(E)-enamido)penicillanic acid</u>

6β-[D,L-4-Chloro-3-thien-2-yl-2-(2,2,2-trichloroethoxy carbonylamino)but-3(E)-enamido]penicillanic acid (0.67g) was deprotected as described in Example 2c. Chromatography on HP20SS gave the <u>title compound</u> isolated as a white freeze dried solid (25mg, 5%); $\nu_{max}$(KBr) 3339, 1774, 1688, 1599, 1511 and 709 $cm^{-1}$; $\delta_H$(250MHz, $D_2O$) 1.43, 1.46 and 1.55 (together 6H, 3s), 4.18 and 4.21 (together 1H, 2s), 5.43 and 5.47 (together 1H, 2d, J 4Hz), 5.55-5.7 (2H, m), and 7.0-7.5 (4H, m). [Mass spectrum: +ve ion (3-NOBA/$Na^+$) $MNa^+$ (438), $\underline{M}Na^+$ (Na salt) (460)].

Example 21

a. Methyl 5-dichloromethyl-5-methyl-2-oxazolin-4-yl-carboxylate

1,1-Dichloroacetone (9.6ml) and methyl isocyanoacetate (6.9ml) were reacted in the presence of triethylamine (12.9ml) using the method described in Example 19b to give the title compound as an oil (17.14g, 84%); $\delta_H$(60MHz, $CDCl_3$) 1.52 (3H, s), 3.67 (3H, s), 4.72 (1H, d, J 2Hz), 5.53 (1H, s), and 6.72 (1H, d, J 2Hz). [Mass spectrum: +ve ion (ammonia) $MH^+$ (226)].

b. Methyl 4-chloro-2-formamido-3-methylbut-3-enoate

Methyl 5-dichloromethyl-5-methyl-2-oxazolin-4-ylcarboxylate was treated with activated zinc dust as described in Example 18b. The crude product was chromatographed on silica to give the Z isomer of the title compound as a colourless oil (3%); (Found: $M^+$, 190.0265. $C_7H_9ClNO_3$ requires M, 190.0271); $\delta_H$ (60MHz, $CDCl_3$) 1.78 (3H, br s), 3.82 (3H, s), 5.75 (1H, d, J 7Hz), 6.12 (1H, br s), 6.87 (1H, br s, exch $D_2O$), and 8.27 (1H, s).

Further elution of the column provided the E isomer of the title compound, which was crystallised from ethyl acetate-hexane (13%), m.p. 103-5°C; (Found: C, 44.00; H, 5.30; N,7.25; Cl. 18.88%. $C_7H_{10}ClNO_3$ requires C, 43.88; H, 5.26; N, 7.31; Cl, 18.50%); $\delta_H$ (250MHz, $CDCl_3$) 1.76 (3H, d, J 3Hz), 3.80 (3H, s), 5.19 (1H, d, J 7.4Hz), 6.32 (1H, m), 6.61 (1H, br s, exch $D_2O$), and 8.23 (1H, s). Confirmed as E isomer by N.O.E.

c. Methyl 2-amino-4-chloro-3-methylbut-3(E)-enoate hydrochloride

Methyl 4-chloro-2-formamido-3-methylbut-3(E)-enoate (1.56g) was hydrolyed as described in Example 18c. The crude produce was triturated with ether to give the title compound as a crystalline solid (1.42g, 88%), m.p. 169-71°C (dec); $\delta_H$ (60MHz, $CD_3OD$) 1.82 (3H, s), 3.83 (3H, s), 4.92 (1H, s), and 6.68 (1H, br s).

d. Methyl 4-chloro-3-methyl-2-(2,2,2-trichloroethoxycarbonylamino)but-3(E)-enoate

Methyl 2-amino-4-chloro-3-methylbut-3(E)-enoate hydrochloride (1.00g) was acylated as described in Example 18d. The title compound was crystallised from hexane (1.06g, 63%), m.p. 79-82°C; (Found: C, 31.67; H, 3.19; N, 4.18; Cl, 41.41%. $C_9H_{11}Cl_4NO_4$ requires C,31.89; H, 3.27; N, 4.13; Cl, 41.83%); $\delta_H$(60MHz, $CDCl_3$) 1.78 (3H, br s),3.80 (3H, s), 4.72 (2H, s), 4.87 (1H, d, J 8Hz), 6.07 (1H, br d, J 8Hz, exch $D_2O$), and 6.32 (1H, br s).

e. 4-Chloro-3-methyl-2-(2,2,2-trichloroethoxycarbonylamino)but-3(E)-enoic acid

Methyl 4-chloro-2-methyl-2-(2,2,2-trichloroethoxycarbonylamino)but-3(E)-enoate (0.51g) was hydrolysed as described in Example 18e to give the title compound as a gum (0.41g, 84%); (Found: $M^+$-Cl, 287.9599, $C_8H_9Cl_3NO_4$ requires $M$, 287.9598; $M^+$-$CO_2H$, 277.9310. $C_7H_8Cl_4NO_2$ requires $M$, 277.9309); $\delta_H$ (60MHz, $CDCl_3$) 1.87 (3H, br s), 4.80 (2H, s), 4.93 (1H, d, J 8Hz), 6.48 (1H, br s), 7.31 (1H, br d, J 8Hz, exch $D_2O$), and 7.82 (1H, br s, exch $D_2O$).

f. 6β-[D,L-4-Chloro-3-methyl-2-(2,2,2-trichloroethoxycarbonylamino)but-3(E)-enamido]penicillanic acid

Prepared as described in example 2b, to give the title compound as the free acid (88%).

g. 6β-(2-Amino-4-chloro-3-methylbut-3(E)-enamido) penicillanic acids

6β-[D,L-4-Chloro-3-methyl-2-(2,2,2-trichloroethoxycarbonylamino)but-3(E)-enamido]penicillanic acid (0.58g) was deprotected as described in Example 2c. The crude product was chromatographed on HP20SS to give the L-stereoisomer of the title compound (52mg, 14%); $\nu_{max}$ 3368, 1766, 1683, 1602 and 1511 $cm^{-1}$; $\delta_H$ (250MHz, $D_2O$) 1.50 (3H, s), 1.58 (3H, s), 1.80 (3H, d, J 1.5Hz), 4.25 (1H, s), 4.65 (1H, s), 5.45 (1H, d, J 4Hz), 5.58 (1H, d, J 4Hz), and 6.59 (1H, br s); followed by a mixture of D and L stereoisomers (26mg, 7%) then the D stereoisomer of the title compound (59mg, 15%); $\nu_{max}$ (KBr) 3382, 1767, 1685, 1602 and 1521 $cm^{-1}$; $\delta_H$(250MHz, $D_2O$) 1.49 (3H, s), 1.59 (3H, s), 1.82 (3H, d, J 1.3Hz), 4.22 (1H, s), 4.70 (1H, s), 5.47 (1H, d, J 3.8Hz), 5.55 (1H, d, J 3.8Hz), and 6.60 (1H, br s); [Mass spectrum: +ve ion (thioglycerol) $MH^+$ (348)].

## Example 22

a. Methyl 2-amino-4-chloro-3-methylbut-3(Z)-enoate hydrochloride

Methyl 4-chloro-2-formamido-3-methylbut-3(Z)-enoate (0.78g, from Example 21b) was hydrolyed as described in Example 18c to give the title compound as a foam (0.68g, 84%); $\delta_H$ (60MHz, $CD_3OD$) 1.88 (3H, br s), 3.90 (3H, s), 5.30 (1H, s), and 6.58 (1H, br s).

b. Methyl 4-chloro-3-methyl-2-(2,2,2-trichloroethoxycarbonylamino)but-3(Z)-enoate

Methyl 2-amino-4-chloro-3-methylbut-3(Z)-enoate hydrochloride (0.68g) was acylated as described in Example 18d. The title compound was crystallised from hexane (0.66g, 58%), m.p. 110-3°; $\delta_H$(60MHz, $CDCl_3$) 1.82 (3H, br s), 3.86 (3H, s), 4.80 (2H, s), 5.62 (1H, d, J 8Hz), 6.18 (1H, br s), and 6.30 (1H, br d, J 8Hz, exch $D_2O$).[Mass spectrum:, $M^+$-Cl (302), $M^+$-$CO_2CH_3$ (278)].

c. 4-Chloro-3-methyl-2-(2,2,2-trichloroethoxycarbonylamino)but-3(Z)-enoic acid

Methyl 4-chloro-3-methyl-2-(2,2,2-trichloroethoxycarbonylamino)but-3(Z)-enoate (0.41g) was hydrolysed as described in Example 18e. The title compound was crystallised from ethyl acetate-hexane (0.21g, 53%), m.p. 132-4°; $\delta_H$(60MHz, $CDCl_3$)1.83 (3H, d, J 1Hz), 4.75 (2H, s), 5.56 (1H, d, J 7Hz), 6.23 (1H, q, J 1Hz), 6.98 (1H, br d, J 7Hz), and 8.07 (1H, br s).

d. 6β-[D,L-4-Chloro-3-methyl-2-(2,2,2-trichloroethoxycarbonylamino)but-3(Z)-enamido]penicillanic acid

Prepared as described in Example 2b to give the title compound, isolated as the free acid (52%).

e. 6β-(D,L-2-Amino-4-chloro-3-methylbut-3(Z)-enamido) penicillanic acid

6β-[D,L-4-Chloro-3-methyl-2-(2,2,2-trichloroethoxycarbonylamino)but-3(Z)-enamido]penicillanic acid (0.54g) was deprotected as described in Example 2c to give the title compound isolated by chromatography on HP20SS, as a white freeze dried solid (0.10g, 28%); $\nu_{max}$ (KBr) 3344, 1771, 1688, 1602, 1514 and 1313$cm^{-1}$;

$\delta_H$ (250MHz, $D_2O$) 1.49, 1.50, 1.57 and 1.60 (together 6H, 4s), 1.79 and 1.80 (together 3H, 2d, J 1.6Hz), 4.24 and 4.25 (together 1H, 2s), 5.20 and 5.21 (together 1H, 2s), 5.47 and 5.52 (together 1H, 2d, J 4Hz), 5.56 and 5.60 (together 1H, 2d, J 4Hz), and 6.45 (1H, br s). [Mass spectrum: +ve ion (thioglycerol) $\underline{M}H^+$ (348)].

Example 23

a. D-2-(2,2,2-Trichloroethoxycarbonylamino)pent--4-enoic acid

D-2-Aminopent-4-enoic acid hydrochloride (2.33g) in aqueous THF at pH 8±1, was acylated with 2,2,2-trichloroethyl chloroformate (2.5ml) using the method described in Example 2a. Flash chromatography gave the title compound as a colourless oil (2.92g, 55%); $\delta_H$ (60MHz, $(CD_3)_2CO$) 2.4-2.8 (2H, m), 4.2-4.6 (1H, m), 4.80 (2H, s), 5.0-5.4 (2H, m), 5.6-6.3 (1H, m), 7.75 (1H, d, J 8Hz), and 8.99 (1H, s).

b. Benzhydryl D-2-(2,2,2-trichloroethoxycarbonylamino)pent-4-enoate

Diphenyldiazomethane (1.63g) in dichloromethane (20ml) was added dropwise over 20 minutes to D-2-(2,2,2-trichloroethoxycarbonylamino)pent-4-enoic acid (2.9g) in dichloromethane. At the end of the addition, all the pink colour had disappeared so further diphenyldiazomethane (0.19g) in dichloromethane was added and then again to give a permanent pink colour. Glacial acetic acid was added to decolourise the solution after 1h. Then the mixture was concentrated *in vacuo* to an oil which solidified on standing overnight. The title compound was obtained from hexane (3.87g, 91%), m.p. 84-87.5°C; $\nu_{max}$ (KBr) 3357, 1743, 1725, 1534, 1270, 1238, 1220, 1107 and 702cm$^{-1}$; $\delta_H$ (60MHz, $(CD_3)_2CO$)

2.4-2.9 (2H, m), 4.3-4.8 (1H, m) 4.82 (2H, s), 4.9-5.4 (2H, m), 5.6-6.3 (1H, m), 6.98 (1H, s), and 7.44 (10H, s).

c. Benzhydryl 3-hydroxy-D-2-(2,2,2-trichloroethoxycarbonylamino)pent-4-enoate

t-Butyl hydroperoxide, 70% aqueous solution (1.85ml) in dichloroethane (ca. 40ml) was dried over $MgSO_4$ and filtered. This solution was added to benzhydryl 2-(2,2,2-trichloroethoxycarbonylamino)pent-4-enoate (3.30g) and selenium dioxide (0.36g). The mixture was stirred at 71°C for 3.5h under reflux, and then stirred at room temperature over a weekend. The liquor was decanted off, partially concentrated in vacuo and the title compound (0.94g, 28%) obtained after flash chromatography on silica gel. Unreacted starting material (2.10g, 64%), was recovered and treated as above to yield the title compound (0.90g, 42%). The overall yield after two passes was 54%. $\delta_H$(60MHz, $(CD_3)_2CO$) 4.7 (2H, s), 4.4-5.5 (4H, m), 5.5-6.3 (1H, m), 6.7 (1H, d, J 9Hz), 6.87 (1H, s), and 7.4 (10H, s).

d. Benzhydryl 3-acetoxy-D-2-(2,2,2-trichloroethoxycarbonylamino)pent-4-enoate

Acetic anhydride (0.06g) was added to benzhydryl 3-hydroxy-D-2-(2,2,2-trichloroethoxycarbonylamino)pent-4-enoate (0.29g) and 4-dimethylaminopyridine (1mg) in pyridine (5ml). The reaction mixture was stirred under argon at room temperature for 6h. The mixture was diluted with water, acidified and extracted with ethyl acetate (2 x 30ml). The combined organic extracts were washed with water (2 x 30ml) and brine, dried and concentrated in vacuo. The title compound was obtained

as a mixture of D- and L- O-acetyl isomers after flash chromatography on silica (0.21g, 67%); $\delta_H$(60MHz, $CDCl_3$) 1.76 and 1.93 (together 3H, 2s), 4.76 (2H, s), 4.6-4.9 (1H, m), 5.1-6.2 (5H, m), 6.95 (1H, s), and 7.36 (10H, s). [Mass spectrum: +ve ion (3-NOBA, $Na^+$) $MNa^+$(536)].

e. Benzhydryl 5-acetoxy-D-2-(2,2,2-trichloroethoxycarbonylamino)pent-3-enoate

Benzhydryl 3-acetoxy-D-2-(2,2,2-trichloroethoxycarbonylamino)pent-4-enoate (1.35g) and bis(acetonitrile)palladium (II) chloride (65mg) in dry THF were stirred for 24h at room temperature, open to the atmosphere. The mixture was concentrated in vacuo and the title compound obtained after flash chromatography on silica (0.85g, 63%); $\delta_H$(60MHz, $CDCl_3$) 1.97 (3H, s), 4.45-4.65 (2H, m), 4.72 (2H, s), 5.1 (1H, br d), 5.8-6.0 (2H, m), 6.1 (1H, d, J 8Hz), 6.97 (1H, s), and 7.33 (10H, s).

f. 5-Acetoxy-D-2-(2,2,2-trichloroethoxycarbonylamino)pent-3-enoic acid

Trifluoroacetic acid (4.5ml) and anisole (0.63ml) were mixed and added to benzhydryl 5-acetoxy-D-2-(2,2,2-trichloroethoxycarbonylamino)pent-3-enoate (0.70g). The mixture was stirred at room temperature for 6 minutes, then diluted with toluene and concentrated in vacuo, this was repeated twice. After flash chromatography, the title compound was obtained by trituration from hexane (0.33g, 69%), m.p. 94.5-97°C; $\nu_{max}$ (KBr) 3413, 3000 (br), 2995, 1734, 1694, 1533, 1403, 1328, 1227, 1196, 1101, 823, 711, and 570$cm^{-1}$; $\delta_H$(60MHz, $CDCl_3$) 2.09 (3H, s), 4.6-5.0 (5H, m), 5.75-6.0 (3H, m), and 8.5 (1H, br s).

g. 6β-[5-Acetoxy-D-2-(2,2,2-trichloroethoxycarbonylamino)pent-3(E)-enamido]penicillanic acid

Oxalyl chloride (0.07g) was added to DMF (0.04g) in dichloromethane (3ml) at -20°C under argon. The mixture was stirred for 15minutes in an ice bath, then cooled to -20°C. 5-Acetoxy-D-2-(2,2,2-trichloroethoxycarbonylamino)pent-3-enoic acid (0.20g) in dichloromethane (3ml) was added. The mixture was stirred for 15 minutes in an ice bath, then cooled to -15°C. 6-Aminopenicillanic acid triethylammonium salt (0.21g) and triethylamine (0.07g) in dichloromethane (3ml) were added and the mixture was stirred at room temperature for 1.1h. The mixture was concentrated *in vacuo*, partitioned between water (15ml) and ethyl acetate (15ml), acidified and separated. The aqueous phase was re-extracted with ethyl acetate (15ml). The combined organic extracts were washed with water (15ml x 2) and brine (15ml), dried ($MgSO_4$) and concentrated in vacuo, to yield the crude title compound (0.33g, quantative).

h. 6β-[5-Acetoxy-D-2-aminopent-3(E)-enamido]penicillanic acid

The title compound was obtained from 6β-[5-Acetoxy-D-2-(2,2,2-trichloroethoxycarbonylamino)pent-3(E)-enamido]penicillanic acid by a procedure similar to that described in Example 2c (0.11g, 52%); $\nu_{max}$ (KBr) 3389, 2969, 1771, 1688, 1602, 1386, 1316 and 1242cm$^{-1}$; $\delta_H$(250MHz, $D_2O$) 1.49 (3H, s), 1.60 (3H, s), 2.12 (3H, s), 4.23 (1H, s), 4.58-4.69 (3H, m), 5.48 (1H, d, J 3.8Hz), 5.55 (1H, d, J 3.8Hz), 5.81-5.91 (1H, m), and 6.14-6.24 (1H, m). [Mass spectrum: +ve ion (thioglycerol) $MH^+$ (372)].

Example 24

a. Methyl 3-bromo-2-methoxyiminopropanoate

Methyl bromopyruvate (43.66g, 0.241mol) and methoxyamine hydrochloride (20.15g, 0.241mol) were dissolved in methanol (220ml) and stirred for 4h at room temperature. After evaporation, the residue was taken up in ethyl acetate, washed with water and brine, dried and evaporated to give a yellow oil (45.0g, 90%). A pure sample was obtained after flash chromatography; $\delta_H$(60MHz, $CDCl_3$) 3.9 (3H, s), 4.17 (3H, s), and 4.21 and 4.38 (2H, 2s).

b. Methyl 3-dimethylphosphonyl-2-methoxyimino-propanoate

Methyl 3-bromo-2-methoxyiminopropanoate (20.00g, 95mmol) and trimethylphosphite (40ml) were refluxed for 1h. The excess trimethylphosphite was removed *in vacuo*, and the residue distilled to give, after a forerun of dimethyl methanephosphonate, the *title compound* as a colourless oil (19.45g, 86%), b.p. 159-161$^oC$/4.5mmHg; $\nu_{max}$ (film) 1720, 1270, 1210, 1170, 1040, 850 and 780$cm^{-1}$; $\delta_H$(250MHz, $CDCl_3$) 3.34 (2H, d, J 23.5Hz), 3.75 (6H, d, J 11.2Hz), 3.89 (3H, s), and 4.13 (3H, s), (single isomer).

c. Methyl 3-cyclohexylidene-2-methoxyiminopropanoate

Methyl 3-dimethylphosphonyl-2-methoxyiminopropanoate (7.17g, 30mmol) in THF (20ml) was added dropwise to a stirred suspension of sodium hydride (50% in oil, 1.44g, 30mmol) in THF (100ml). The mixture was stirred for 2h, then cyclohexanone (3.11ml, 30mmol) in THF (15ml) added dropwise. The mixture was stirred overnight, then diluted with ammonium chloride solution

(150ml), and extracted with ethyl acetate (3x50ml). The combined extracts were washed with brine, dried and evaporated. Flash chromatography on silica gel, using 0-5% ethyl acetate in hexane as eluant, gave the title compound as a colourless oil (2.31g, 36%); $\nu_{max}$ (film) 2930, 1730, 1430, 1200, 1150, 1050 and 760cm$^{-1}$; $\delta_H$(250MHz, $CDCl_3$) 1.5-2.4 (10H, m), 3.85 (3H, s), 4.05 (3H, s), and 5.77 (1H, s), (single isomer).

d. Methyl 2-amino-3-cyclohexylidenepropanoate

Prepared as described in Example 12b (49%); $\nu_{max}$ (film) 2930, 2850, 1735, 1440, 1430, 1200 and 1170cm$^{-1}$; $\delta_H$(60MHz, $CDCl_3$), 1.4-2.4 (12H, m), 3.7 (3H, s), 4.3 (1H, d, J 9Hz), and 5.1 (1H, d, J 9Hz).

e. Methyl 3-cyclohexylidene-2-(2,2,2-trichloroethoxycarbonylamino)propanoate

Prepared as described in Example 12c (quantitative); $\delta_H$(60MHz, $CDCl_3$) 1.4-1.8 (6H, m), 2.0-2.5 (4H, m), 3.75 (3H, s), 4.75 (2H, s), 5.0-5.3 (2H, m), and 5.93 (1H, br).

f. 3-Cyclohexylidene-2-(2,2,2-trichloroethoxycarbonylamino)propanoic acid

Prepared as described in Example 12d, using only one equivalent of base (56%), m.p. 139-140°C (ethyl acetate/cyclohexane); $\nu_{max}$ (KBr) 3334, 2937, 1706, 1526, 1292, 1240, 1052 and 723cm$^{-1}$; $\delta_H$(250MHz, $(CD_3)_2CO$) 1.58 (6H, m), 2.14 (2H, m), 2.33 (2H, m), 4.77 and 4.82 (2H, ABq, J 12.2Hz), 5.03 (1H, dd, J 9.1, 7.6Hz), 5.23 (1H, d, J 9.1Hz), and 7.14 (1H, br d, exch $D_2O$).

g. 6β-[D,L-3-Cyclohexylidene-2-(2,2,2-trichlorethoxy-carbonylamino)propanamido]penicillanic acid

Prepared as described in Example 2b, but not converted to the sodium salt (92%).

h. 6β-(2-Amino-3-cyclohexylidenepropanamido) penicillanic acids

Deprotection of 6β-[D,L-3-cyclohexylidene-2-(2,2,2-trichloroethoxycarbonylamino)propanamido]penicillanic acid (1.6g, 2.9mmol) was performed as described in Example 2c. Chromatography of the crude product on HP20SS, eluting with 6-7% THF in water, provided 6β-(L-2-amino-3-cyclohexylidenepropanamido)penicillanic acid (105mg, 10%); $\nu_{max}$ (KBr) 2929, 2855, 1757, 1710, 1606, 1511 and 1322cm$^{-1}$; $\delta_H$(250MHz, $D_2O$) 1.5-1.7 (12H, m), 2.1-2.45 (4H, m), 4.61 (1H, s), 4.96 (1H, d, J 10.0Hz), 5.23 (1H, d, J 10.0Hz), 5.51 (1H, d, J 4.1Hz), and 5.69 (1H, d, J 4.1Hz); followed by a mixture of D- and L- stereoisomers (163mg, 15%); then 6β-(D-2-amino-3-cyclohexylidenepropanamido)penicillanic acid (90mg, 8%); $\nu_{max}$ (KBr) 2926, 1766, 1701, 1608, 1512, 1396 and 1325cm$^{-1}$; $\delta_H$(250MHz, $D_2O$) 1.45-1.7 (12H, m), 2.15-2.4 (4H, m), 4.56 (1H, s), 4.90 (1H, d, J 10.0Hz), 5.19 (1H, d, J 10.0Hz), 5.52 (1H, d, J 4.0Hz), and 5.62 (1H, d, J 4.0Hz).

**Example 25**

**Methyl 2-hydroxyimino-4-methoxy-3-oxobutanoate**

Sodium nitrite (11.64g) in water (45ml) was added dropwise over 0.5h to an ice bath cooled solution of methyl 4-methoxyacetoacetate (21.9g) in glacial acetic acid (30ml), below 20°C. The mixture was stirred for a further 0.25h, then cooling was removed. After a

further 0.25h the mixture was diluted with water and extracted with ethyl acetate (4x50ml). The combined organic extracts were washed with water (2x50ml), 1M $NaHCO_3$ solution (3x50ml) and brine (2x50ml). The aqueous phases were back extracted with ethyl acetate. The organic phase was dried, concentrated and crystallised from ethyl acetate/hexane (19.7g, 75%), m.p. 109-112$^{o}$C; (Found: C, 41.30; H, 4.99; N, 7.95%. $C_6H_9NO_5$ requires C, 41.15; H, 5.18; N, 8.00%); $\nu_{max}$ (KBr) 3213, 1755, 1704, 1384, 1030 and 1007cm$^{-1}$; $\delta_H$(60MHz, $(CD_3)_2CO$) 3.38 (3H, s), 3.84 (3H, s), 4.54 (2H, s), and 11.81 (1H, s).

Methyl 4-methoxy-2-methoxyimino-3-oxobutanoate

Dimethyl sulphate (10ml) in acetone (20ml) was added dropwise over 0.25h to an ice bath cooled solution of methyl 2-hydroxyimino-4-methoxy-3-oxobutanoate (19.27g) and potassium carbonate (15.20g) in acetone (80ml). The cooling was removed and the mixture stirred at room temperature for 4.5h. Ethyl acetate (100ml) and water (100ml) were added to the partially concentrated solution. The aqueous phase was extracted again with ethyl acetate. The combined organic extracts were washed with water and brine, dried and concentrated *in vacuo*. Flash chromatography, eluting with 10-25% ethyl acetate/hexane, yielded the title compound (10.66g, 51%); $\nu_{max}$ (film) 1752, 1715, 1439, 1309, 1221 and 1049cm$^{-1}$; $\delta_H$(60MHz, $CDCl_3$) 3.44 (3H, s), 3.87 (3H, s), 4.10 (3H, s), and 4.54 (2H, s).

Methyl 4-methoxy-2-methoxyimino-3-methylenebutanoate

Methyltriphenylphosphonium bromide (14.16g) was added in portions to a suspension of potassium t-butoxide (4.45g) in THF (50ml) at room temperature under argon.

The mixture was heated at reflux for 1h, cooled to room temperature, and methyl 4-methoxy-2-methoxyimino-3-oxo-butanoate (5.1g) in THF (35ml) added. The mixture was heated at 60°C for 5 minutes, then cooled. Water (50ml) and ethyl acetate (50ml) were added, and the aqueous phase extracted again with ethyl acetate (50ml). The combined organic extracts were washed with water (2x50ml), brine (2x50ml), dried and concentrated in vacuo. Flash chromatography, eluting with 10% ethyl acetate/hexane, yielded the title compound (2.35g, 47%); $\nu_{max}$ (film) 1747, 1343, 1243, 1088 and 1041cm$^{-1}$; $\delta_H$(60MHz, $CDCl_3$) 3.35 (3H, s), 3.80 (3H, s), 3.87 (3H, s), 4.0-4.2 (2H, m), 5.2-5.3 (1H, m), and 5.6-5.7 (1H, m).

Methyl 2-amino-4-methoxy-3-methylenebutanoate

Prepared as described in Example 12b (70%); $\nu_{max}$ (film) 2900 (br), 1750, 1580 (br), 1240 and 1100cm$^{-1}$; $\delta_H$(60MHz, $CDCl_3$) 3.30 (3H, s), 3.78 (3H, s), 4.05 (2H, s), 4.59 (1H, s), 5.3-5.6 (2H, m), and 8.6-9.0 (2H, br).

Methyl 4-methoxy-3-methylene-2-(2,2,2-trichloroethoxy-carbonylamino)butanoate

Prepared as described in Example 12c (81%), m.p. 50-52°C (hexane); $\nu_{max}$ (KBr) 3407, 1735, 1501, 1322, 1203 and 1089cm$^{-1}$; $\delta_H$(60MHz, $CDCl_3$) 3.32 (3H, s), 3.77 (3H, s), 4.01 (2H, s), 4.76 (2H, s), 4.97 (1H, d, J 6.7Hz), 5.36 (2H, s), and 6.1 (1H, br).

4-Methoxy-3-methylene-2-(2,2,2-trichloroethoxycarbonylamino)butanoic acid

Prepared as described in Example 12d (35%); $\nu_{max}$ (KBr) 3390, 1744, 1714, 1699, 1514 and 1223cm$^{-1}$; $\delta_H$(60MHz, $(CD_3)_2CO$) 3.29 (3H, s), 4.05 (H, s), 4.79 (2H, s), 4.91 (1H, d, J 8.7Hz), 5.2-5.5 (2H, m), 7.0 (1H, br), and 8.45 (1H, br).

6β-[D,L-4-Methoxy-3-methylene-2-(2,2,2-trichloroethoxycarbonylamino)butanamido]penicillanic acid

Prepared as described in Example 2b, but not converted to the sodium salt (88%).

6β-(D,L-2-Amino-4-methoxy-3-methylenebutanamido) penicillanic acid

Deprotection of 6β-[D,L-4-methoxy-3-methylene-2-(2,2,2-trichloroethoxycarbonylamino)butanamido]penicillanic acid (0.71g) as described in Example 2c gave the title compound, isolated after chromatography on HP20SS, as a white freeze dried solid (0.11g, 23%); $\nu_{max}$ (KBr) 3220, 1775, 1689, 1610, 1524, 1391, 1316 and 1095cm$^{-1}$; $\delta_H$(250MHz, $D_2O$) 1.54 and 1.55 (together 3H, 2s), 1.63 and 1.64 (together 3H, 2s), 3.39 and 3.40 (together 3H, 2s), 4.13 and 4.14 (together 2H, 2s), 4.27 and 4.28 (together 1H, 2s), 4.78 and 4.79 (together 1H, 2s), and 5.5-5.7 (4H, m).

Example 26

a. Methyl 2-methoxyimino-4-methylpent-3-enoate

Prepared as described in Example 24c, using an excess of acetone, to give, after chromatography, the title compound (24%); (Found: $M^+$, 171.0893. $C_8H_{13}NO_3$ requires M, 171.0895); $\nu_{max}$ (film) 1730, 1440, 1300, 1200, 1150, 1060 and 1040cm$^{-1}$; $\delta_H$ (60MHz, $CDCl_3$) 1.62 (3H, s), 1.9 (3H, s), 3.84 (3H, s), 4.02 (3H, s), and 5.9 (1H, m).

b. Methyl 2-amino-4-methylpent-3-enoate

Prepared as described in Example 12b; $\delta_H$ (60MHz, $CDCl_3$) 1.75 (6H, s), 2.1 (2H, s), 3.72 (3H, s), 4.3 (1H, d, J 9Hz), and 5.2 (1H, m).

c. Methyl 4-methyl-2-(2,2,2-trichloroethoxycarbonyl-amino)pent-3-enoate

Prepared as described in Example 12c (quantitative); $\delta_H$ (60MHz, $CDCl_3$) 1.75 (3H, s), 1.80 (3H, s), 3.7 (3H, s), 4.7 (2H, s), 5.0 (2H, m), and 6.0 (1H, br).

d. 4-Methyl-2-(2,2,2-trichloroethoxycarbonylamino)pent-3-enoic acid

Prepared as described in Example 12d, using only one equivalent of base (34% over three steps), m.p. 199-200$^o$C (ethyl acetate/cyclohexane); $\nu_{max}$ (KBr) 3297, 1733, 1701, 1541, 1288, 1250 and 721cm$^{-1}$; $\delta_H$ (250MHz, $(CD_3)_2CO$) 1.77 (3H, d, J 1.1Hz), 1.82 (3H, d, J 1.1Hz), 4.78 and 4.82 (2H, ABq, J 12.2Hz), 4.97 (1H, dd, J 9.1, 7.7Hz), 5.28 (1H, dt, J 9.1, 1.1Hz), and 7.15 (1H, br d).

e. 6β-[D,L-4-Methyl-2-(2,2,2-trichloroethoxycarbonylamino)pent-3-enamido]penicillanic acid

Prepared as described in Example 2b, but not converted to the sodium salt (87%).

f. 6β-(2-Amino-4-methylpent-3-enamido)penicillanic acids

Deprotection of 6β-[D,L-4-methyl-2-(2,2,2-trichloroethoxycarbonylamino)pent-3-enamido]penicillanic acid (1.70g, 3.4mmol) was performed as described in Example 2c. Chromatography of the crude product on HP20SS, eluting with 2% THF in water, provided 6β-(L-2-amino-4-methylpent-3-enamido)penicillanic acid (38mg, 3.4%); $\nu_{max}$ (KBr) 2972, 1774, 1689, 1607, 1517, 1389 and 1315cm$^{-1}$; $\delta_H$ (250MHz, $D_2O$) 1.56 (3H, s), 1.62 (3H, s), 1.89 (6H, s), 4.31 (1H, s), 4.90 (1H, d, J 10.0Hz), 5.30 (1H, m), 5.49 (1H, d, J 4.0Hz), and 5.65 (1H, d, J 4.0Hz); followed by a mixture of D- and L-stereoisomers (276mg, 25%); then 6β-(D-2-amino-4-methylpent-3-enamido) penicillanic acid (77mg, 7%); $\nu_{max}$ (KBr) 2970, 1774, 1690, 1604, 1517, 1390 and 1316cm$^{-1}$; $\delta_H$ (250MHz, $D_2O$) 1.55 (3H, s), 1.63 (3H, s), 1.88 (6H, d, J 1.0Hz), 4.29 (1H, s), 4.90 (1H, d, J 10.0Hz), 5.30 (1H, m), 5.52 (1H, d, J 3.9Hz), and 5.60 (1H, d, J 3.9Hz).

Example 27

The following Table gives the Absorption Index [that is the ratio of urine recoveries from oral (p.o) and subcutaneous (s.c) administration] following administration of the compounds of the invention to mice (5 mice per group) at a dose of 50mg/kg. Comparative figures for Amoxycillin are also given.

Table 1 Absorption Index in the Mouse

| Compound of Example | % Recovery (mean) p.o | s.c | Absorption Index (p.o/s.c) |
|---|---|---|---|
| (1) | 79 | 98 | 0.8 |
| (4) (D-stereoisomer) | 21 | 86 | 0.3 |
| (5) (D-stereoisomer) | 37 | 87 | 0.4 |
| (6) | 44 | 103 | 0.4 |
| (9) (D-stereoisomer) | 34 | 116 | 0.3 |
| (11) (D-stereoisomer) | 48 | 63 | 0.76 |
| (13) (D-stereoisomer) | 45 | 61 | 0.74 |
| (14) (D-stereoisomer) | 11 | 18 | 0.6 |
| Amoxycillin | 23 | 56 | 0.4 |

Example 28

The following Table gives mean Squirrel Monkey Plasma levels in μg/ml following oral administration of 25mg/kg of a compound of the invention to the number of animals indicated.

Comparative figures for Amoxycillin, administered at the same dose, are also given.

Table 2 Squirrel Monkey Plasma Levels.

| Compound of Example No. | Time in Minutes 15 | 30 | 60 | 120 | 240 | 360 | Area under the curve (μg.h/ml) | No.of animals |
|---|---|---|---|---|---|---|---|---|
| (1) | 4.0 | 14.7 | 32.7 | 15.7 | 2.4 | 0.8 | 59.7 | 3 |
| (5) (D-stereoisomer) | 20.7 | 28.8 | 24.3 | 16.8 | 2.4 | 1.4 | 63.0 | 3 |
| (6) | 8.7 | 16.4 | 20.0 | 13.0 | 2.2 | 0.9 | 46.5 | 6 |
| Amoxycillin | 4.8 | 9.7 | 12.6 | 6.7 | 1.1 | 0.3 | 26.2 | 6 |

Example 29

The following Table gives Minimum Inhibitory Concentration (MIC) values for some of the compounds of the invention against S.pyogenes CN 10 and E.coli NCTC 10418. The E. coli was grown in tryptone soya broth and the S. pyogenes was grown in Todd Hewitt broth. The microorganisms were grown for 24hours and 1µl samples were then inoculated on to agar plates (comprising 5% v/v live horse blood agar) to which had been added the compounds of the invention at a series of two-fold dilutions. After a further 24 hours, the MIC values were determined by visual inspection.

Table 3 MIC Data

| Compound of Example | S. pyogenes CN 10 | E. coli NCTC 10418 |
|---|---|---|
| (1) | 0.03 | 8 |
| (4) (D-stereoisomer) | <0.03 | 8 |
| (5) (D-stereoisomer) | <0.03 | 2 |
| (6) | <0.03 | 2 |
| (7) | 0.25 | 32 |
| (8) | 2 | |
| (9) (D-stereoisomer) | <0.03 | 2 |
| (10) (D-stereoisomer) | <0.06 | 2 |
| (11) (D-stereoisomer) | <0.06 | 8 |
| (12) (D-stereoisomer) | 0.06 | 2 |
| (13) (D-stereoisomer) | <0.06 | 4 |
| (14) (D-stereoisomer) | <0.06 | 16 |
| (15) (D-stereoisomer) | <0.06 | 16 |
| (16) | <0.06 | 16 |

Table 3 (Cont'd) MIC Data

| Compound of Example | S. pyogenes CN 10 | E. coli NCTC 10418 |
| --- | --- | --- |
| (17) (D-stereoisomer) | <0.06 | 4 |
| (18) (D-stereoisomer) | 0.06 | 16 |
| (19) | <0.03 | 16 |
| (20) | 0.06 | 32 |
| (21) (D-stereoisomer) | 0.06 | 4 |
| (22) | 0.12 | 8 |
| (23) | 0.12 | 32 |

Example 30

The following Table gives $CD_{50}$ values obtained after infecting groups of mice with a lethal inoculum of various bacteria and treating the mice with the compounds quoted. The $CD_{50}$ value then represents the amount of compound necessary to protect 50% of the animals.

Table 4. $CD_{50}$ Data

| | | Total $CD_{50}$ (mg/kg) | |
|---|---|---|---|
| Organism | Route | Compound of Example 5 (D-stereoisomer) | Compound of Example 6 |
| *S.aureus* Smith | p.o. | 0.05 | 0.08 |
| | s.c. | 0.08 | 0.15 |
| | p.o. | 0.25 | 0.10 |
| | s.c. | 0.09 | 0.15 |
| | (MIC(µg/ml) | 0.06 | 0.12) |
| *S.agalactiae* 2798 | p.o. | 0.6 | 3.8 |
| | s.c. | 0.12 | 1.1 |
| | p.o. | 0.9 | 3.6 |
| | s.c. | 0.5 | 1.5 |
| | (MIC(µg/ml) | 0.06 | 0.25) |
| *E.coli* 96 R- | p.o. | 40 | 32 |
| | s.c. | 22 | 6 |
| | p.o. | 40 | 40 |
| | s.c. | 40 | 28 |
| | (MIC(µg/ml) | 2 | 4) |

Claims

1. A compound of formula (I) or a pharmaceutically acceptable salt or *in vivo* hydrolysable derivative thereof:

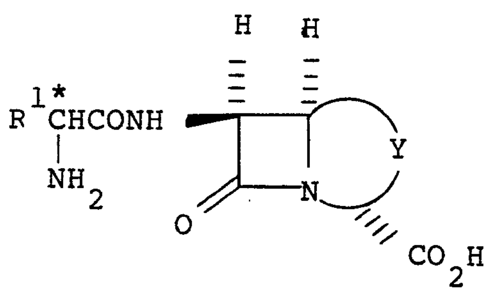

(I)

wherein $R^1$ represents a group:

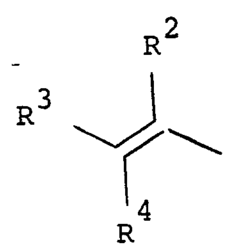

in which $R^2$,$R^3$ and $R^4$ are the same or different and each is hydrogen; halogen; optionally substituted $C_{1-6}$ alkyl; optionally substituted $C_{3-7}$ cycloalkyl; optionally substituted $C_{2-6}$ alkenyl; optionally substituted $C_{2-6}$ alkynyl; amido; cyano; carboxy; formyl; $C_{1-6}$ alkoxycarbonyl; mono- or di-$(C_{1-6})$alkylamido; $C_{1-6}$ alkylcarbonyl; an aryl group; a heterocyclyl group; or $R^3$ and $R^4$ are joined together to form the residue of a carbocyclic ring having a total of from 3 to 7 carbon atoms in the ring; or $R^3$ and $R^4$ are joined together to form the residue of a heterocyclic ring in which $R^3$ and $R^4$ together may be

represented by the formula $-(CH_2)_mX(CH_2)_n-$in which X is oxygen, sulphur or a group $NR^5$ wherein $R^5$ is hydrogen, acyl, an amino-protecting group, or $C_{1-6}$ alkyl, m is 1 to 3 and n is 1 to 3; and Y is:

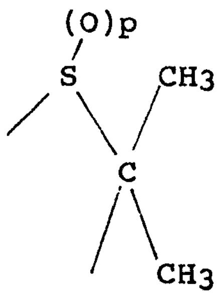

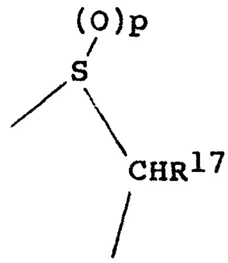

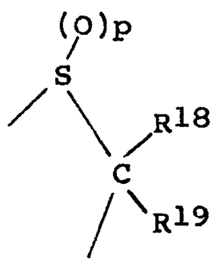

or

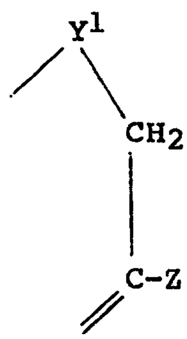

;

wherein $R^{17}$ is hydrogen or $C_{1-6}$ alkyl; $R^{18}$ and $R^{19}$ may be the same or different each being $C_{2-6}$ alkyl; p is an integer having the value 0, 1 or 2; $Y^1$ is oxygen, sulphur, SO, $SO_2$, or $-CH_2-$; and Z represents hydrogen, halogen or an organic group such as $C_{1-4}$ alkoxy, $-CH_2Q$ or $-CH=CH-Q$ wherein Q represents hydrogen, halogen, hydroxy, mercapto, cyano, carboxy, carbamoyloxy, carboxylic ester, $C_{1-4}$ alkyloxy, acyloxy, aryl, a heterocyclyl group bonded via carbon, a heterocyclylthio group or a nitrogen containing heterocyclic group bonded via nitrogen; with the proviso that when Y is $-S-C(CH_3)_2-$ and $R^2$ is methyl, $R^3$ cannot be hydrogen when $R^4$ is phenyl and $R^3$ cannot be phenyl when $R^4$ is hydrogen.

2. A compound as claimed in Claim 1 in which Y is $-S-C(CH_3)_2-$ or $-S-CH_2-CZ=$.

3. A compound as claimed in Claim 1 or Claim 2 in which Y is $-S-C(CH_3)_2-$.

4. A compound as claimed in any one of claims 1-3 in which $R^2$ is hydrogen or $C_{1-6}$ alkyl; and $R^3$ and $R^4$ are the same or different and each is halogen, $C_{1-6}$ alkyl or together form a carbocyclic ring containing 5 or 6 carbon atoms.

5. A compound as claimed in claim 3 in which $R^2$ is hydrogen and $R^3$ and $R^4$ are the same or different and each is halogen or $C_{1-6}$ alkyl.

6. A compound as claimed in claim 3 in which $R^2$ is hydrogen and $R^3$ and $R^4$ together form a carbocyclic ring containing 5 or 6 carbon atoms.

7. A compound as claimed in any one of claims 1 to 6 selected from the following or a pharmaceutically acceptable salt or in vivo hydrolysable ester thereof:

6β-(D-2-Aminobut-3-enamido)penicillanic acid

6β-(D-2-Amino-3-phenylbut-3-enamido)penicillanic acid

6β-(D-2-Amino-4,4-dichlorobut-3-enamido) penicillanic acid

6β-(D-2-Amino-3-methylbut-3-enamido)penicillanic acid

7β-(D-2-Amino-3-methylbut-3-enamido) cephalosporanic acid

7β-[D-2-Amino-3-methylbut-3-enamido]-3-desacetoxy cephalosporanic acid

6β-(D-2-Amino-3-ethylbut-3-enamido) penicillanic acid

6β-(D-2-Amino-3-methylpent-3(E)-enamido)-penicillanic acid

6β-(D-2-Amino-pent-3(E)-enamido)penicillanic acid

6β-(D-2-Amino-3-methylhex-3(E),5-dienamido)-penicillanic acid

6β-(D-2-Aminohex-3(E)-enamido)penicillanic acid

6β-(D-2-Amino-4-cyclohexylbut-3(E)-enamido)-penicillanic acid

6β-(D-2-Amino-4-phenylbut-3(E)-enamido)-penicillanic acid

6β-[D,L-2-Amino-4-(4-chlorophenyl)but-3(E)enamido] penicillanic acid

6,β-(D-2-Amino-3-trifluoromethylbut-3-enamido)-penicillanic acid

6β-(D-2-Amino-3-thien-2-ylbut-3-enamido)-penicillanic acid

6β-(D,L-2-Amino-4-chloro-3-thien-2-ylbut-3(Z)-enamido)penicillanic acid

6B-(D,L-2-Amino-4-chloro-3-thien-2-ylbut-3(E)-enamido)penicillanic acid

6β-(D-2-Amino-4-chloro-3-methylbut-3(E)-enamido)-penicillanic acid

6β-(D,L-2-Amino-4-chloro-3-methylbut-3(Z)-enamido) penicillanic acid

6β-[5-Acetoxy-D-2-aminopent-3(E)-enamido]-penicillanic acid

6β-(D-2-Amino-3-cyclohexylidenepropanamido) penicillanic acid

6β-(D,L-2-Amino-4-methoxy-3-methylenebutanamido) penicillanic acid

6β-(D-2-Amino-4-methylpent-3-enamido) penicillanic acid

8. A pharmaceutical composition comprising a compound as claimed in any one of claims 1 to 7 together with a pharmaceutically acceptable carrier or excipient.

9. A pharmaceutical composition as claimed in claim 8 which further comprises a β-lactamase inhibitor.

10. A process for the preparation of a compound as claimed in Claim 1 which process comprises reacting a compound of formula (III):

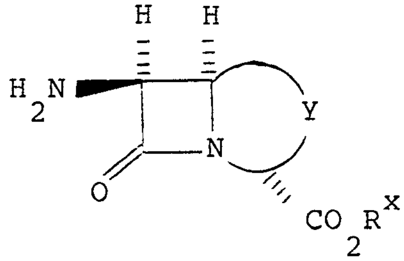

(III)

wherein the amino group is optionally substituted with a group which permits acylation to take place, and $R^x$ is hydrogen or a carboxyl-blocking group, with an N-acylating compound formed from a salt or N-protected derivative of an amino acid of formula (IV):

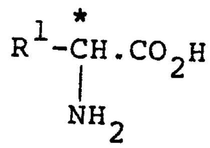

(IV)

wherein $R^1$ and * are as defined with respect to formula (I) above and any reactive groups may be protected; and thereafter, if necessary, carrying out one or more of the following steps:

(i) removing any carboxyl-blocking groups $R^x$;

(ii) removing any protecting groups elsewhere in in the molecule;

(iii) converting the product into a salt or <u>in vivo</u> hydrolysable derivative thereof.

11. A compound as claimed in claim 1, which compound is the <u>in vivo</u> hydrolysable derivative of formula (Ia) or a pharmaceutically acceptable salt or <u>in vivo</u> hydrolysable ester thereof:

 

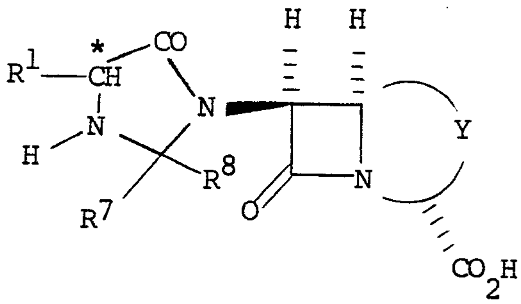

(Ia)

wherein $R^1$, * and Y are as defined with respect to formula (I) and $R^7$ and $R^8$ are the same or different and each is hydrogen or $C_{1-6}$ alkyl.

12. A compound of the formula (IVA):

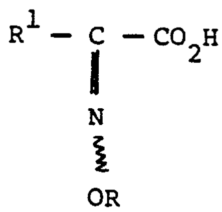

(IVA)

wherein $R^1$ is defined with respect to formula (I) and R is hydrogen, $C_{1-6}$ alkyl, $C_{2-6}$ alkenyl, $C_{3-7}$ cycloalkyl or aryl $C_{1-6}$ alkyl.

13. A process for the preparation of a compound of formula (IV):

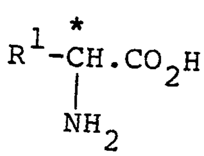

(IV)

wherein $R^1$ is defined with respect to formula (I); which process comprises treating a compound of the formula (IVA):

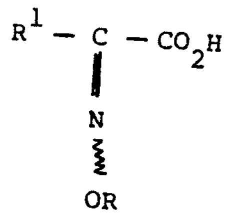

(IVA)

wherein $R^1$ is as hereinbefore defined and R is hydrogen, $C_{1-6}$ alkyl, $C_{2-6}$ alkenyl, $C_{3-7}$ cycloalkyl or aryl $C_{1-6}$ alkyl; with a reducing agent capable of converting the group

H<br>
|<br>
-C=N-OR into the group $-C-NH_2$<br>
| |

whilst leaving the remainder of the molecule unaffected.

14. A process for the preparation of a compound of the formula (IVB) or a salt or <u>in</u> <u>vivo</u> hydrolysable derivative thereof:

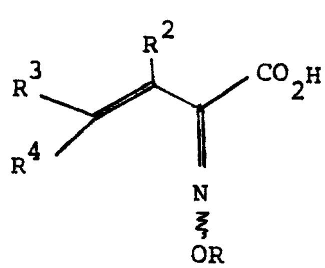

(IVB)

which process comprises:

a) reacting a compound of the formula:

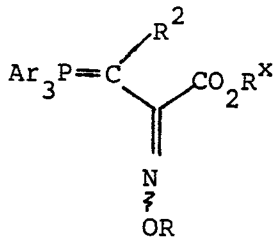

wherein Ar is aryl, $R^2$ is defined as in claim 1, R is as defined in claim 12, and $R^x$ is a carboxyl-blocking group; with a compound of the formula:

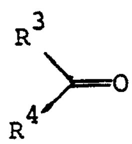

wherein $R^3$ and $R^4$ are as defined in claim 1; or

b) reacting a compound of the formula:

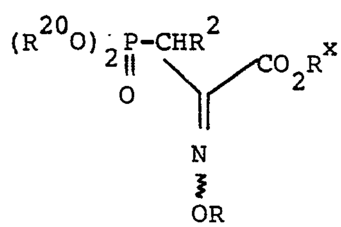

wherein R, $R^2$, and $R^x$ are as hereinbefore defined and $R^{20}$ is $C_{1-6}$ alkyl; with a compound of the formula:

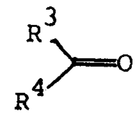

wherein $R^3$ and $R^4$ are as defined in claim 1; or

c) reacting a compound of the formula:

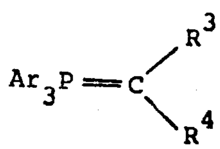

wherein Ar, $R^3$ and $R^4$ are as hereinbefore defined; with a compound of the formula:

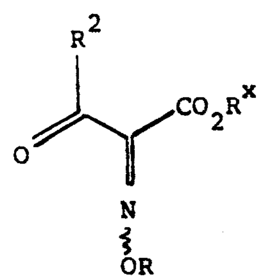

wherein R, $R^2$ and $R^x$ are as hereinbefore defined; and, if necessary, after any of the processes a), b) or c) above, carrying out one or more of the following steps:

i) removing the carboxyl-protecting group $R^x$;

ii) converting the product into a salt or <u>in vivo</u> hydrolysable derivative thereof.

15. The use of a compound of formula (I) as defined in Claim 1 or a pharmaceutically acceptable salt or *in vivo* hydrolysable derivative thereof for the manufacture of a medicament for therapeutic purposes.